(19) Europäisches Patentamt
European Patent Office
Office européen des brevets

(11) Publication number: **0 159 533**
**A2**

# (12) EUROPEAN PATENT APPLICATION

(21) Application number: **85103158.3**

(22) Date of filing: **19.03.85**

(51) Int. Cl.[4]: **C 07 D 285/00**, C 07 D 417/12, A 61 K 31/54, C 07 C 161/00

(30) Priority: **27.03.84 GB 8407853**
**27.07.84 GB 8419224**
**27.07.84 GB 8419223**

(43) Date of publication of application: **30.10.85**
**Bulletin 85/44**

(84) Designated Contracting States: **AT BE CH DE FR GB IT LI LU NL SE**

(71) Applicant: **HOECHST U.K. LIMITED, Hoechst House Salisbury Road, Hounslow Middlesex TW4 6JH (GB)**

(72) Inventor: **Ross, Barry Clive, Dr., 41 Blandford Avenue, Luton Bedfordshire (GB)**
Inventor: **Michael, Jeffrey Daniel, 5 Wood Street Woburn Sands, Milton Keynes Buckinghamshire (GB)**
Inventor: **Cousins, Simon John, 1 Gleman Close Greenleys, Milton Keynes Buckinghamshire (GB)**

(74) Representative: **Beck, Bernhard et al, HOECHST AKTIENGESELLSCHAFT Zentrale Patentabteilung Postfach 80 03 20, D-6230 Frankfurt/Main 80 (DE)**

(54) **Thiatriazine derivatives.**

(57) Compounds of formula I

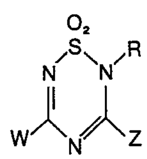

I

are valuable H-2 antagonists; R represents hydrogen, alkyl, phenyl or phenalkyl; W and Z represent groups E-$(CH_2)_n$-X-$(CH_2)_m$-NH with E being phenyl, imidazolyl, thiazolyl, furyl, thienyl or pyridyl; or W or Z is hydrogen, alkyl, phenyl, O-alkyl, $NR^4R^5$ ($R^4/R^5$ are hydrogen, alkyl or phenyl); X is O or S; n is 0 or 1, m is 2, 3 or 3.

Moreover compounds XXXI are described

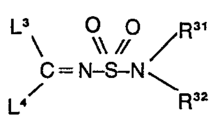

XXXI

in which $R^{31}/R^{32}$ are H, alkyl, cycloalkyl, phenyl, phenylkyl or part of 4-to 7-membered ring: $L^3/L^4$ are X-(alkyl-phenyl, -phenalkyl), compounds XXXI are versatile intermediates for the production of i.a. compounds I.

Compounds XXXV

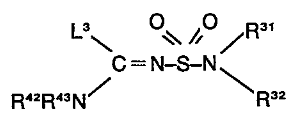

XXXV

with $R^{42}/R^{43}$ being hydrogen, alkyl, phenyl, phenyalkyl are also versatile intermediates inter alia for the preparation of compounds I.

Processes for the preparation of these compounds are disclosed as well.

EP 0 159 533 A2

ACTORUM AG

The present invention relates to thiatriazine derivatives which have histamine H-2 antagonist activity. The invention also relates to processes for the preparation of these derivatives, to pharmaceutical preparations comprising them, and to their use.

Histamine is one of a number of naturally occurring physiologically active substances which are thought to interact with specific receptors. In the case of histamine, there are at least two types: one is called the H-1 receptor (Ash and Schild, Brit. J. Pharmac. 1966, 27, 427), and the other is called the H-2 receptor (Black et al Nature 1972, 236, 385). The action of histamine at the H-1 receptor, for example, stimulation of bronchial and gastro-intestinal smooth muscle, is blocked by the compounds generally known as "antihistamines", but which are now also called histamine H-1 antagonists, for example, mepyramine. The action of histamine at the H-2 receptors, for example, stimulation of gastric acid secretion and heart rate, is not blocked by mepyramine but is blocked by other compounds, for example, burimamide and cimetidine.

Histamine H-2 antagonists may be used to treat those conditions resulting from stimulation by histamine of H-2 receptors, either alone, for example, in inhibiting gastric acid secretion and thus treating its sequelae, for example, gastric and peptic ulcers; or together with H-1 antagonists, for example, in allergic and certain inflammatory conditions.

The present invention provides compounds of formula I, which are histamine H-2 antagonists:

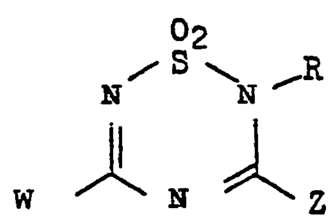

I

in which formula

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

W and Z, which may be the same or different, each represents a group $E-(CH_2)_n - X -(CH_2)_m - NH-$ in which E represents a phenyl, imidazolyl, thiazolyl, furyl, thienyl, or pyridyl radical,

which radicals may be unsubstituted or may have one or two substitutents, selected from straight and branched chain alkyl groups having from 1 to 4 carbon atoms, guanidino and mono-alkyl guanidino groups, the alkyl moiety of which has a straight or branched chain and from 1 to 4 carbon atoms, and groups of the formula II

$$- Q - N \begin{matrix} R^1 \\ R^2 \end{matrix} \qquad \text{II}$$

in which Q represents an alkylene group having from 1 to 4 carbon atoms, and $R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl substitutent having from 1 to 4 carbon atoms, or

one of W and Z represents a group E - $(CH_2)_n$ - X - $(CH_2)_m$ - NH- as defined above, and the other represents a hydrogen atom; a straight or branched chain alkyl group having from 1 to 6 carbon atoms; a phenyl group; an $-OR^3$ group in which $R^3$ represents a straight or branched chain alkyl group having from 1 to 4 carbon atoms; or an $NR^4R^5$ group in which $R^4$ and $R^5$, which may be the same or different, each represents a hydrogen atom, an unsubstituted or substituted straight or branched chain alkyl group having from 1 to 6 carbon atoms, or a phenyl group;

X represents -O- or -S-;

n represents 0 or 1; and

m represents 2, 3 or 4.

This invention also provides salts of a compound of formula I, especially physiologically tolerable salts.

Alkyl groups in the present specification may be branched or unbranched, and alkyl groups, for example, alkyl groups $R^4$ and $R^5$, may be unsubstituted or substituted, for example, by one or more, for example, one or two, groups selected from

(i) $-OR^6$ groups in which $R^6$ represents a straight or branched chain alkyl group having from 1 to 6 carbon atoms;

(ii) cycloalkyl groups having from 3 to 7 carbon atoms;

(iii) $-NR^7R^8$ groups in which $R^7$ and $R^8$, which may be the same or different, each represents a hydrogen atom, a straight or branched chain alkyl group having from 1 to 4 carbon atoms, a phenyl group or an acyl group having from 1 to 4 carbon atoms in the alkyl moiety, and $R^7$ and $R^8$ together with the nitrogen atom to which they are attached may form a 5 or 6 membered ring;

(iv) $-COOR^9$ groups in which $R^9$ represents a straight or branched chain alkyl group having from 1 to 4 carbon atoms;

(v) $-CONR^7R^8$ groups in which $R^7$ and $R^8$ are defined above;

(vi) alkylsulphonyl groups having from 1 to 4 carbon atoms

in the alkyl moiety and phenylsulphonyl groups;

(vii) cyano and nitro groups;

(viii) phenyl groups which may be substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl moiety having a straight or branched chain and from 1 to 4 carbon atoms;

(ix) aromatic and non-aromatic heterocyclic groups having from 5 to 8 ring members and one or two hetero-atoms selected from oxygen, sulphur and nitrogen atoms, and optionally having an alkyl substitutent having from 1 to 4 carbon atoms on a ring nitrogen atom, for example, a furyl, tetrahydrofuryl, thienyl, pyridyl, dihydropyranyl, pyrrolidinyl, N-lower alkyl-pyrrolidinyl, or piperidyl group.

A phenyl group other than a phenyl group E (which is as defined above), may be unsubstituted or substituted as defined in (viii) above.

It will be understood that the structure given above for formula I is only one of several possible tautomeric and isomeric representations. The present invention includes all possible tautomeric and isomeric forms of compounds of the general formula I.

In the present specification the term "lower" is used to denote a group having from 1 to 4 carbon atoms. The term "halogen" denotes chlorine, bromine, iodine and fluorine. The term "known" is used herein to mean in actual use in the art or described in the literature of the art.

It will be appreciated by a worker skilled in the art that a combination of substitutents that are incompatible for steric reasons or because of potential inter-reactions should not be chosen. The worker will also use his normal discretion in the number of substitutents chosen.

The present invention also provides a process for the production of a compound of formula I, which comprises reacting a 1,2,4,6-thiatriazine of formula IIIA or IIIB

IIIA 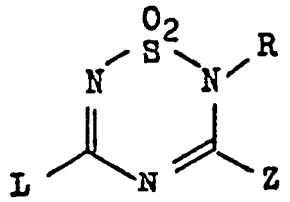 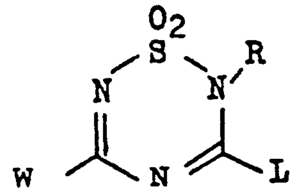 IIIB

in which R, W and Z are as defined above, W and L having any of the meanings given above, and L represents a halogen atom, an alkoxy, phenoxy or phenylalkoxy group, an alkylthio, phenylthio or phenylalkylthio group, an alkylsulphinyl, phenylsulphinyl or phenalkylsulphinyl group, or an alkylsulphonyl, phenylsulphonyl or phenylalkylsulphonyl group, in which groups an alkyl group or alkyl moiety has a straight or branched chain and from 1 to 4 carbon atoms, and a phenyl group may be substituted as defined in (viii) above, with a compound of the general formula IV

$$E-(CH_2)_n-X-(CH_2)_m-NH_2 \qquad IV$$

in which E, X, m and n are as defined for formula I, and, if desired, carrying out any one or more of the following reactions in any desired order:-

(i) converting a group R into another group R,

(ii) converting an acid addition salt of formula I into the corresponding free base or converting a free base into an acid addition salt, or

(iii) in a resulting compound of formula I in which one of the groups D and Z represents a group $NR^4R^5$, converting this group into another group $NR^4R^5$.

In addition to serving as a substituent in the final product, $R^4$ may also act as a protecting group in the above chemical transformations and may subsequently be removed, for example, by hydrogenolysis or by acid or base catalysed hydrolysis to provide a compound of the general formula I in which W or Z is a group $-NHR^5$ or $-NH_2$.

Protecting groups which come within the definition of $R^4$ are t-butyl and benzyl groups, for example.

Compound IV is generally reacted with compound IIIA or with compound IIIB in a solvent or diluent, preferably in an alcohol, acetone, acetonitrile or DMF. The reaction temperature is, for example, within the range of from 0 to $80^{o}$ C, depending on the nature of the leaving group L. Generally when L is a halogen atom, an alkylsulphinyl group or a substituted phenoxy group, the reaction occurs rapidly at O to $20^{o}$C, but when L is an alkylthio or alkoxy group, elevated reaction temperatures, for example, in the range of from 50 to 100°C, for example, from 60 to 80°C are preferred.

The compound of formula IV is reacted in the form of the free base, as shown. If it is initially present in the form of an acid addition salt, for example, as the hydrochloride or hydrobromide, this is converted into the free base during or, preferably, before reaction with compound IIIA or IIIB. Conversion is carried out with an organic or inorganic base, for example, sodium hydroxide or potassium hydroxide, or a tertiary amine, for example, triethylamine.

A compound of formula I may be converted into its salt form in the usual manner by reaction with an acid. Examples of physiologically tolerable acid addition salts are those with hydrobromic, hydrochloric, sulphuric, acetic, malonic, maleic, fumaric, succinic, citric, tartaric and isethionic acids.

A compound of formula I in the form of an acid addition salt may be converted into the free base form by reaction with a base in the usual manner.

A number of compounds of the type represented by formula IV are known, see, for example, GB Specification 2,001,624A; U.S. Patent 3,950,333; U.S. Patent 4,128,658; and Belgian Patents 867,106 and 875,846, and the others may be prepared analogously.

Some examples of the thiatriazine intermediates

represented by formula IIIB are disclosed in U.S. patent 4,013,447, DE-OS 2,943,703, DE-OS 3,134,143, DE-OS 2,933,889, and EPA 0,029,908.

However, many of the specific intermediates of formula IIIB and all of the compounds of formula IIIA have not been described previously, and form part of the present invention. Some thiatriazines of the general formula IIIA may be prepared as shown in Scheme I.

Scheme I

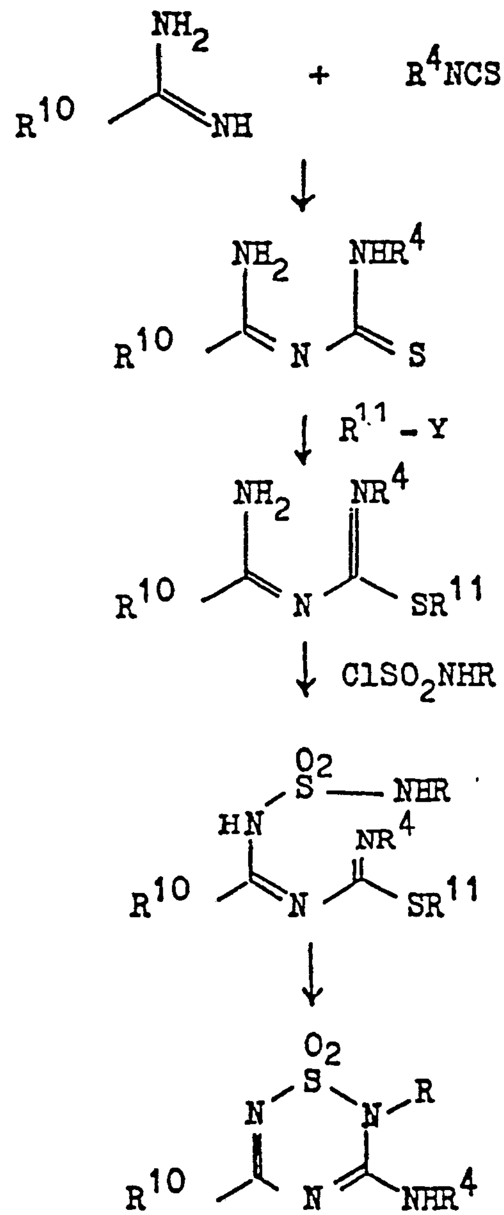


V

VI

VII

VIII

IX

X

XI

XII

in which R and $R^4$ are as defined above;

$R^{10}$ represents an alkoxy, phenoxy, phenylalkoxy, alkylthio, phenylthio or phenylalkylthio group as defined above for L , for example, a methoxy, benzyloxy or methylthio group; and

$R^{11}$-Y represents an alkylating agent, for example, $R^{11}$ represents an alkyl or phenalkyl group, for example, a lower alkyl group or a phenyl-lower alkyl group, for example, a methyl or benzyl group, and Y represents a halogen atom, for example, an iodine or bromine atom. $R^{11}$-Y represents, for example, methyl iodide or benzyl bromide. When $R^{11}$ is different from and distinguishable from $R^{10}$, the relative positions of R and $R^4$NH- on the thiatriazine ring of compound XII can be distinguished.

A compound V is reacted with an isothiocyanate VI in an inert solvent, for example, acetonitrile or a chlorinated hydrocarbon, for example, methylene chloride, preferably at a temperature within the range of from 20 to 50°C, to give a compound of formula VII, which is alkylated with an alkylating agent VIII, preferably at a temperature in the range of from 20 to 50°C and generally in a solvent, for example, acetonitrile, an ether, for example, THF or, preferably, an alcohol, for example, methanol or ethanol, to give the compound of formula IX. Compound IX is then reacted with an alkylaminosulphonyl chloride of formula X, in the presence of a tertiary base, for example, triethylamine, to give compound XI. The reaction is generally carried out in a solvent, for example, acetonitrile, an ether, or a chlorinated hydrocarbon, for example, methylene chloride, preferably at a temperature of from 0 to 20°C. Compound XI is cyclised by heating, for example, at a temperature within the range of from 60 to 160°C, preferably from 60 to 80°C, in an aprotic solvent, for example, dioxane, acetonitrile or diglyme, preferably in the presence of an organic base, for example, a tertiary base, for example, triethylamine, to give the thiatriazine of formula XII. Alkylaminosulphonyl chlorides of formula X are known, see for example, J. Org. Chem., 41, 4028 (1976).

Other thiatriazines of general formula IIIA may be prepared as shown in Scheme II

Scheme II

XIII

X

XIV

XV

XVI

XVII

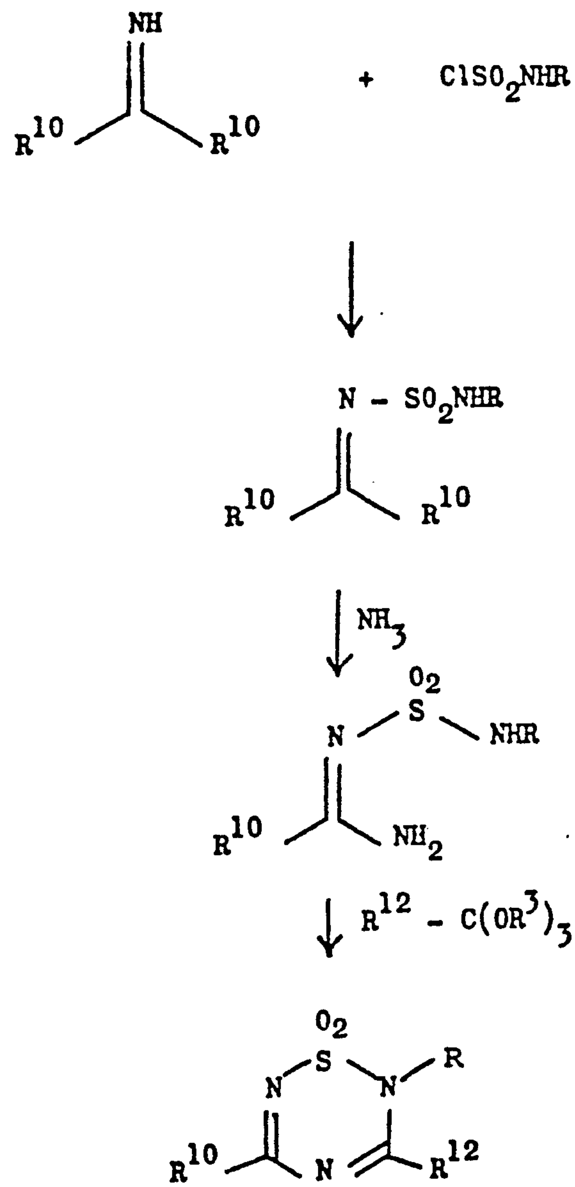


in which R and $R^{10}$ are as defined above, and $R^{12}$ represents a hydrogen atom, a straight or branched chain alkyl group having from 1 to 6 carbon atoms, a phenyl group or an $-OR^3$ group, in which $R^3$ represents a lower alkyl group, especially a methyl or ethyl group. An alkyl or phenyl group may be substituted as defined above.

A compound of formula XIII is reacted with an alkylaminosulphonyl chloride of formula X, preferably in the presence of a tertiary base, for example, triethylamine, to give compound XIV. The reaction is generally carried out in a solvent, for example, acetonitrile, an ether, or a chlorinated hydrocarbon, for example, methylene chloride, preferably at a temperature in the range of from 0 to 20°C. Compound XIV is generally reacted with one equivalent of anhydrous ammonia, preferably in an inert solvent, for example, tetrahydrofuran or dioxane, preferably at a temperature in the range of from 0 to 20°C to give compound XV, which is cyclised to the thiatriazine XVII by reaction with ortho ester XVI. This reaction may be carried out using an excess of the ortho ester as solvent or, preferably, using an inert solvent, for example, acetonitrile, tetrahydrofuran, dioxane or diglyme, preferably at an elevated temperature, for example, from 80 to 160°C.

Imidocarbonates and dithioimidocarbonates of formula XIII are known, see for example, Z. Chem. 8, 459-460, (1968), Beilstein E113 page 156, and Bull. inst. recherches biol. unic. Perm, 6, (10), 517-522, (1929).

Thiatriazines of formula XII or formula XVII in which $R^{10}$ represents an alkylthio, phenylthio or phenylalkylthio group may be converted to other compounds of formula IIIA, for example, by oxidation of the thio group to the corresponding sulphinyl or sulphonyl group, or by replacement of the alkylthio, phenylthio or phenylalkylthio group by a halogen atom, especially a chlorine atom, for example, as shown in Scheme III, in which $Y^1$ represents a halogen atom, for example, a chlorine atom, $R^{13}$ represents an alkyl, phenyl or phenylalkyl group, for example, a lower alkyl, phenyl or benzyl group, and x represents 1 or 2.

Scheme III

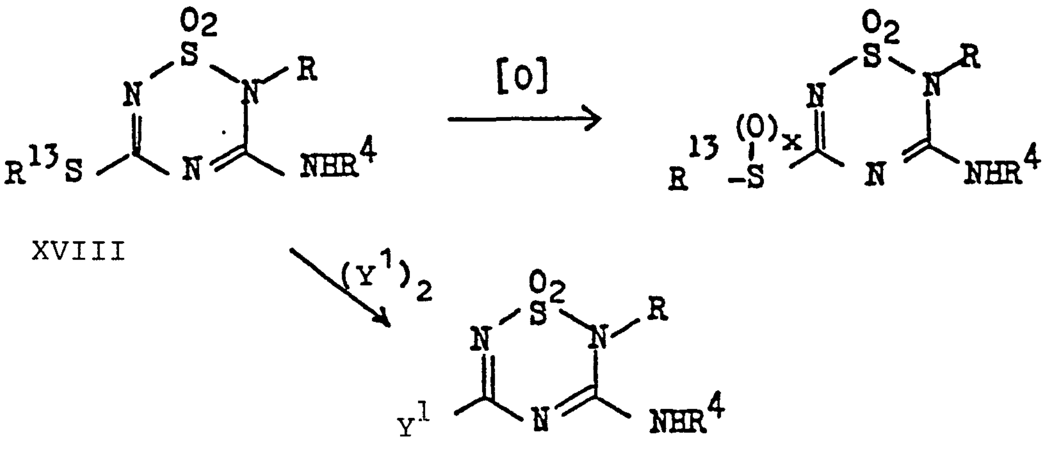


XVIII

XIX

XX

Such a conversion may be carried out by known methods, for example, by oxidising the sulphide group using, for example, a peracid, for example, m-chloroperbenzoic acid; hydrogen peroxide; or a periodate, perborate or permanganate salt. Replacement of an alkylthio or arylthio group by a halogen atom may be carried out, for example, by treating compound XVIII with excess chlorine or bromine, for example, by treatment with elemental chlorine or bromine, or with a compound capable of yielding the desired halogen, for example, phosphorus pentachloride. The halogenation is generally carried out in an inert solvent, for example, a chlorinated hydrocarbon, an ether or an ester, for example, chloroform, tetrahydrofuran or ethyl acetate, generally at a temperature within the range of from 20 to 100°C, and preferably in the presence of a catalyst, for example, anhydrous zinc chloride.

Certain thiatriazines of formula IIIA and of formula IIIB may be prepared from a dichlorothiatriazine of formula XXII as shown below in Scheme IV, in which R, $R^4$, $R^5$ and $R^{13}$ are as defined above:

Scheme IV

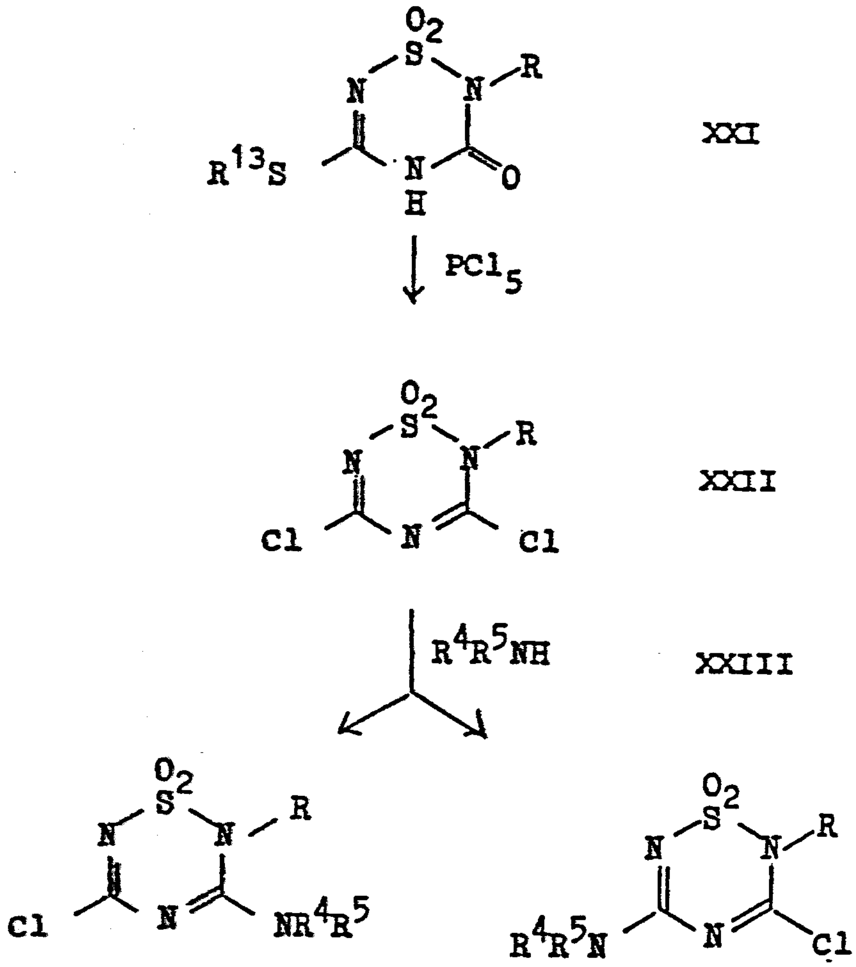


XXI

XXII

XXIII

XXIV

XXV

Treatment of a compound of formula XXI (which is known, see for example, DE-OS 2,943,703), with excess phosphorus pentachloride gives the dichlorothiatriazine of formula XXII. This reaction is carried out in an inert solvent, for example, a chlorinated hydrocarbon, for example, 1,2-dichloroethane, or phosphorus oxychloride, generally at a temperature within the range of from 80 to 105°C. Compound XXII is then reacted with a primary or secondary amine XXIII in an aprotic solvent, for example, diethyl ether, dichloromethane or acetonitrile, generally at a temperature within the range of from -40 to +20°C to give a mixture of isomers XXIV and XXV, which can be separated by known methods, for example, by fractional

crystallisation or by chromatography. Higher temperatures, for example, from 0 to 20°C generally give a mixture of the two isomers, whereas lowering the temperature of the reaction between compounds XXII and XXIII to within the range of from -40 to -20°C gives predominantly the isomer of formula XXIV. Isomers XXIV and XXV fall within the general formulae IIIA and IIIB respectively. Compounds of the formulae XI, XII, XIV, XV, XVII, XVIII, XIX, XX, XXII, XXIV and XXV are new and form part of the present invention (see also below for compounds XIV and XV).

The present invention also includes all possible tautomeric and stereoisomeric forms of those compounds of the invention that can form such isomers.

Examples of preferred compounds of formula I are the following:

(1) R = methyl, Z = methylamino, W = E-$(CH_2)_n$ - X -$(CH_2)_m$ - NH- in which E = [2-[(2-Guanidino-4-thiazolyl), n = 1, X = S, and m = 2 i.e.
3-[2-[(2-Guanidino-4-thiazolyl)methylthio]ethyl]amino-5-methylamino-6-methyl-1,2,4,6-thiatriazine-1,1-dioxide.

(2) R = methyl, Z = tert.-butylamino, W = E-$(CH_2)_n$ - X -$(CH_2)_m$ - NH- in which E = [2-[(2-Guanidino-4-thiazolyl), n = 1, X = S, and m = 2 i.e.
[2-[(2-Guanidino-4-thiazolyl)methylthio]ethyl]amino-5-tert.-butylamino-6-methyl-1,2,4,6-thiatriazine-1,1-dioxide.

(3) R = methyl, Z = tert.-butylamino, W = E-$(CH_2)_n$ - X -$(CH_2)_m$ - NH- in which E = [3-[3-(1-Piperidinylmethyl-phenyl), n = 0, X = O, and m = 3 i.e.
3-[3-[3-(1-Piperidinylmethyl)phenoxy]propyl]amino-5-tert.-butylamino-6-methyl-1,2,4,6-thiatriazine-1,1- dioxide.

(4) R = methyl, W = tert.butylamino, Z = E-$(CH_2)_n$ - X -$(CH_2)_m$ - NH- in which E = [2-[(2-Guanidino-4-thiazolyl), n = 1, X = S, and m = 2 i.e.
2-Methyl-3-[2-[(2-guanidino-4-thiazolyl)methylthio]-ethyl]-amino-5-tert.-butylamino-1,2,4,6-thiatriazine-1,1-dioxide and the hydrochloride salt thereof.

(5) R = methyl, W = dimethylamino, Z = E-$(CH_2)_n$ - X -$(CH_2)_m$ - NH- in which E = [2-[(2-Guanidino-4-thiazolyl), n = 1, X = S, and m = 2 i.e.
2-Methyl-3-[2-[(2-guanidino-4-thiazolyl)methylthio]-ethyl]amino-5-dimethylamino-1,2,4,6-thiatriazine-1,1-dioxide.

(6) R = methyl, W = methoxy, Z = E-$(CH_2)_n$ - X -$(CH_2)_m$ - NH- in which E = [2-[(2-Guanidino-4-thiazolyl), n = 1, X = S, and m = 2 i.e.
2-Methyl-3-[2-[(2-guanidino-4-thiazolyl)methylthio]-ethyl]amino-5-methoxy-1,2,4,6-thiatriazine-1,1-dioxide.

(7) R = methyl, W = phenyl, Z = E-$(CH_2)_n$ - X -$(CH_2)_m$ - NH- in which E = [2-[(2-Guanidino-4-thiazolyl), n = 1, X = S, and m = 2 i.e.
2-Methyl-3-[2-[(2-guanidino-4-thiazolyl)methylthio]-ethyl]amino-5-phenyl-1,2,4,6-thiatriazine-1,1-dioxide.

(8) R = methyl, Z = amino, W = E-$(CH_2)_n$ - X -$(CH_2)_m$ - NH- in which E = [2-[(2-Guanidino-4-thiazolyl), n = 1, X = S, and m = 2 i.e.
3-[2-[(2-Guanidino-4-thiazolyl)methylthio]ethyl]amino-5-amino-6-methyl-1,2,4,6-thiatriazine-1,1- dioxide, and the trifluoroacetic acid salt thereof.

(9) R = methyl, W = amino, Z = E-$(CH_2)_n$ - X -$(CH_2)_m$ - NH- in which E = [2-[(2-Guanidino-4-thiazolyl), n = 1, X = S, and m = 2 i.e.
2-Methyl-3-[2-[(2-guanidino-4-thiazolyl)methylthio]-ethyl]amino-5-amino-1,2,4,6-thiatriazine-1,1-dioxide.

(10) R = methyl, Z = amino, W = E-$(CH_2)_n$ - X -$(CH_2)_m$ - NH- in which E = [3-[3-(1-Piperidinylmethylphenyl), n = 0, X = O, and m = 3 i.e.
3-[3-[3-(1-Piperidinylmethyl)phenoxy]propyl]amino-5-amino-6-methyl-1,2,4,6-thiatriazine-1,1-dioxide, and its trifluoroacetic acid salt.

(11) R = methyl, W = amino, Z = E-$(CH_2)_n$ - X -$(CH_2)_m$ - NH- in which E = [3-[3-(1-Piperidinylmethylphenyl), n = 0, X = O, and m = 3 i.e.

2-Methyl-3-[3-[3-(1-piperidinylmethyl)phenoxy]-propyl]amino-5-amino-1,2,4,6-thiatriazine-1,1-dioxide, and its trifluoroacetic acid salt.

The present invention further provides a pharmaceutical preparation which comprises a compound of the general formula I or a physiologically tolerable salt thereof as active ingredient, in admixture or conjunction with a pharmaceutically suitable carrier. The preparation may be in a form suitable for enteral or parenteral administration, for example, for oral or intravenous administration. The preparation may be in unit dosage form, for example, as tablets or capsules, or in unit or multiple dose ampoules or vials. From 0.1 to 10 mg of the active substance may be administered per kg body weight. A preparation of the invention may also comprise one or more pharmaceutically active substances, for example, histamine H-1 antagonists.

The present invention also provides a compound of the general formula I for use as a medicament, especially for use as a histamine H-2 antagonist, and further provides a method of treating conditions resulting from stimulation by histamine of H-2 receptors, either alone, for example, in inhibiting gastric acid secretion and thus treating its sequelae, for example, gastric and peptic ulcers, or together with H-1 antagonists, for example, in treating allergic and certain inflammatory conditions.

The present invention further provides the use of a compound of formula I for the manufacture of a medicament for the treatment of conditions arising from stimulation by histamineof histamine H-2 receptors.

The present invention also provides compounds of formula XXXI

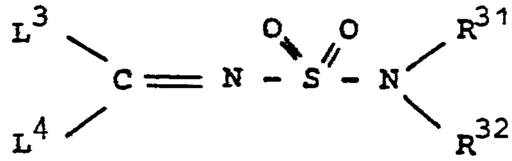

XXXI

in which

$R^{31}$ and $R^{32}$, which may be the same or different, each represents a hydrogen atom, a straight or branched chain alkyl group having from 1 to 4 carbon atoms, a cycloalkyl group having from 3 to 7 carbon atoms, a phenyl group, or a phenalkyl group having from 1 to 4 carbon atoms in the alkyl moiety,

wherein an alkyl, cycloalkyl, phenyl or phenalkyl group may be unsubstituted or substituted by one or more substitutents, which may be the same or different, for example, by one or two substitutents, especially by one substitutent, selected from halogen atoms, straight or branched chain alkyl groups having from 1 to 4 carbon atoms, straight or branched chain alkoxy groups having from 1 to 4 carbon atoms, and nitro and trifluoromethyl groups; or

$R^{31}$ and $R^{32}$ together with the nitrogen atom to which they are attached, form a 4- to 7-membered ring which may contain one or more further hetero-atoms, for example, selected from nitrogen, oxygen and sulphur atoms;

$L^3$ and $L^4$, which may be the same or different, each represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two, and especially by one substitutent, selected from alkyl, alkoxy, methylenedioxy, phenoxy,

halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms,

with the proviso that in $L^3$ and $L^4$, the two radicals $R^{46}$ may be the same or different but the two radicals X must be the same, and

with the exception of those compounds in which $R^{31}$ and $R^{32}$ both represent hydrogen atoms and $L^3$ and $L^4$ both represent alkylthio or alkoxy groups.

Compounds of formula XXXI may exist in various isomeric and tautomeric forms. The present invention includes all isomeric and tautomeric forms of a compound of formula XXXI.

In the present specification, the term "lower" denotes groups having up to 4 carbon atoms, and any alkyl group can have a straight or branched chain.

In a compound of formula XXXI, $R^{31}$ preferably represents a hydrogen atom, and $R^{32}$ preferably represents a hydrogen atom or a lower alkyl group, especially a methyl group or an ethyl group. Particularly preferred are those compounds in which $R^{31}$ represents a hydrogen atom and $R^{32}$ represents a methyl group and especially preferred are those compounds in which $R^{31}$ and $R^{32}$ both represent hydrogen atoms.

When X in $L^3$ and $L^4$ represents a sulphur atom, $L^3$ preferably represents a lower alkylthio group, especially a methylthio group, and $L^4$ preferably represents a phenylthio group. When X represents an oxygen atom, $L^3$ and $L^4$ both preferably represent substituted phenoxy groups, especially mono-substituted phenoxy groups, for example, nitro-substituted phenoxy groups, for example, orthonitrophenoxy groups.

The following compounds of formula XXXI are particularly preferred:

(1) $R^{31} = R^{32}$ = hydrogen, $L^3$ = methylthio and $L^4$ = phenylthio,

i.e. S-Methyl,S'-phenyl-N-sulphamoyldithioimidocarbonate (2) $R^{31}$ = hydrogen, $R^{32}$ = methyl, $L^3$ = methylthio and $L^4$ = phenylthio, i.e. S-Methyl,S'-phenyl-N-(N'-methyl)-sulphamoyldithioimidocarbonate; (3) $R^{31}$ = $R^{32}$ = hydrogen, $L^3$ = $L^4$ = 2-nitrophenyloxy ie Bis-(2-nitrophenyl)-N-sulphamoylimidocarbonate; and (4) $R^{31}$ = hydrogen, $R^{32}$ = methyl, $L^3$ = $L^4$ = 2-nitrophenyloxy ie Bis-(2-nitrophenyl)-N-(N'-methyl)-sulphamoylimidocarbonate.

The present invention provides a process for the production of a compound of formula XXXI as defined above, that is to say, including those compounds in which $R^{31}$ and $R^{32}$ both represent hydrogen atoms and $L^3$ and $L^4$ represent alkylthio or alkoxy groups, which comprises reacting a compound of the general formula XXXII

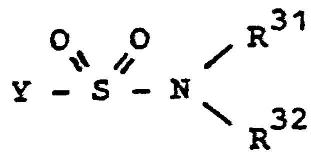

XXXII

in which Y represents a halogen atom, especially a chlorine atom, and $R^{31}$ and $R^{32}$ are as defined above, with a compound of the general formula XXXIII or with a compound of the general formula XXXIV

XXXIII 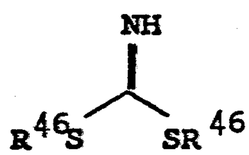

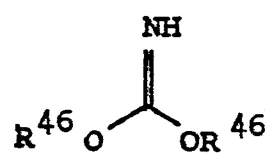 XXXIV

in which $R^{46}$ is as defined above.

A compound of formula XXXII may be prepared as described in Chem. Ber. 91, 1339, (1958), in DE-OS 937,645, J. Org. Chem. 41 4028 (1976) or in J. Med. Chem. 15 (5) 538 (1972), and is preferably used when freshly prepared. A compound of formula XXXIII may be prepared as described in Z. Chem. 8 (12) 459-460 (1968), Bull. inst. recherches biol unic. Perm, 6, (10), 517-522, or Beilstein E113 page 156, and a compound of formula XXXIV as described in Liebig's Annalen 287 310, 319 and 321 (1895).

In a compound of formula XXXIII, one radical $R^{46}$ preferably represents a methyl group and the other preferably represents a phenyl group. In a compound of formula XXXIV, both radicals $R^{46}$ preferably represent substituted phenyl groups, for example, ortho-nitrophenyl groups.

The reaction between the compound of formula XXXII and the compound of formula XXXIII or XXXIV is preferably carried out in the presence of an organic base, especially a tertiary amino base, for example, triethylamine.

The reaction is preferably carried out in the presence of an aprotic solvent, for example, acetonitrile, or an ether, an aromatic hydrocarbon, a chlorinated aliphatic or aromatic hydrocarbon, for example, diethyl ether, tetrahydrofuran, benzene or dichloromethane.

The reaction is preferably carried out under an inert atmosphere, for example, under nitrogen. The reaction is generally carried out at a temperature within the range of from -50 to +80°C, peferably from -5 to +45°C.

Preferably, a compound of formula XXXII, advantageously freshly prepared, is added dropwise to a solution of the compound of formula XXXIII or XXXIV, and the base is preferably added simultaneously with the compound of formula XXXII. This addition may be carried out over a period of from 5 minutes to 8 hours, preferably from 10 minutes to 2 hours.

Compounds of formula XXXI in which $R^{31}$ and $R^{32}$ both represent hydrogen atoms and $L^3$ and $L^4$ both represent alkylthio or both represent alkoxy groups are disclosed in UK Patent Specification No. 1 398 426, which discloses a different process which results in the production of those compounds of formula XXXI in which $R^{31}$ and $R^{32}$ both represent hydrogen atoms, and $L^3$ and $L^4$ both represent alkylthio groups only.

Compounds of formula XXXI are versatile intermediates which can be used in the production of a wide range of sulphamoyl guanidines, sulphamoyl isothioureas, sulphamoyl

thioureas, and a variety of heterocyclic systems by displacement of the leaving group $L^3$ and/or the leaving group $L^4$ by a suitable group. For example, by reacting a compound of formula XLVII

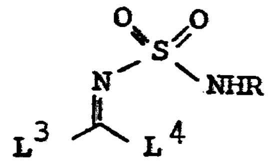

XLVII

in which $L^3$ and $L^4$ are as defined above and R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

wherein an alkyl, phenyl or phenyalkyl group may be unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

with an amine of formula XLVI

$$HNR^{42}R^{43} \qquad XLVI$$

in which $R^{42}$ and $R^{43}$ are as defined for R, and R, $R^{42}$ and $R^{43}$ may be the same or different, there is produced a compound of formula XLIII

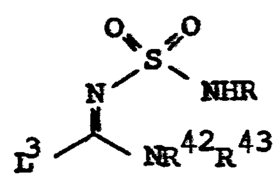

XLIII

in which $L^3$, R, $R^{42}$ and $R^{43}$ are as defined above.

A compound of formula XLIII may, in its turn, be reacted with a compound of formula XLV

$$A - (CH_2)_n - X - (CH_2)_m - NH_2 \qquad XLV$$

in which

A represents a phenyl or furyl radical,

which radical may be unsubstituted or may have one or more, especially one or two, substitutents which may be the same or different, selected from guanidino and mono-alkyl guanidino groups, the alkyl moiety of which has a straight or branched chain and from 1 to 4 carbon atoms, and from groups of formula II

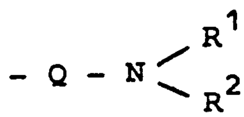 II

in which Q represents an alkylene group having from 1 to 4 carbon atoms, and $R^1$ and $R^2$, which may be the same or different, each represents a hydrogen atom, or a straight or branched chain alkyl group having from 1 to 4 carbon atoms, or $R^1$ and $R^2$ together with the nitrogen atom to which they are attached form a 4 to 8-membered ring which may contain an oxygen atom or another nitrogen atom, which nitrogen atom may have an alkyl group having from 1 to 4 carbon atoms as substitutent, or

A represents a thiazolyl or thiadiazolyl radical that is substituted by one or more, especially by one or two, substituents which may be the same or different and which are as defined above for substitutents of phenyl and furyl groups A;

X represents an oxygen or sulphur atom;

n represents 0 or 1; and

m represents 2, 3 or 4 to give a compound of formula XLI

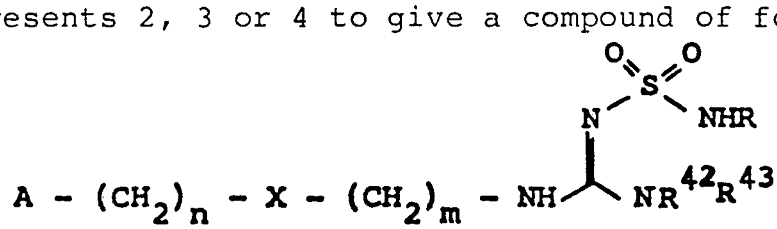 XLI

in which A, X, n, m, R, $R^{42}$ and $R^{43}$ are as defined above.

The reaction between compounds XLVII and XLVI, and between compounds XLIII and XLV, respectively, is generally carried out in a solvent, for example, an organic solvent, for example, a lower alcohol, for

example, methanol, ethanol or isopropanol, dimethylformamide, tetrahydrofuran or acetonitrile, or in a mixture of two or more of such solvents. The reaction is generally carried out at a temperature within the range of from 5 to 75°C, especially at room temperature.

Compounds of formula XLI and processes for their production are described and claimed in our co-pending application no.           (Case 19). Compounds analogous to compounds XLI may be prepared analogously from other compounds of formula XXXI.

An example of the use of a compound of formula XXXI in the production of a heterocyclic system is as follows:

A compound of formula XIV

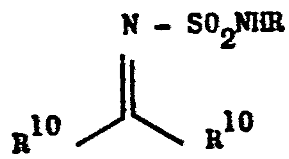

XIV

in which R is as defined above, and $R^{10}$ represents an alkoxy, phenoxy, phenylalkoxy, alkylthio, phenylthio or phenylalkylthio group wherein a phenyl or phenalkyl group may be unsubstituted or substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl moiety having a straight or branched chain and from 1 to 4 carbon atoms, is reacted with ammonia to give a compound of formula XV

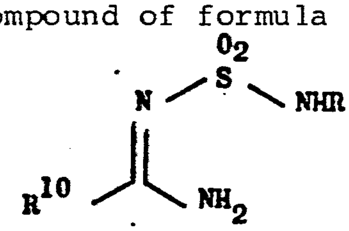

XV

in which R and $R^{10}$ are as defined above. Compound XV is then reacted with a compound of formula XVI

$$R^{12} - C(OR^3)_3 \qquad XVI$$

in which $R^3$ represents a straight or branched chain alkyl

group having from 1 to 4 carbon atoms, and $R^{12}$ represents a hydrogen atom, an alkyl group having from 1 to 6 carbon atoms, a phenyl group or an $-OR^3$ group, in which $R^3$ is as defined above, to give a compound of formula XVII

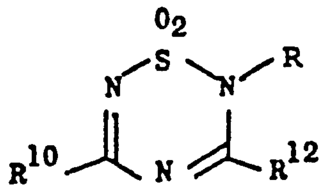

XVII

in which R, $R^{10}$ and $R^{12}$ are as defined above.

A compound of formula XVII may then be reacted with a compound of formula IV

$$E-(CH_2)_n-X-(CH_2)_m-NH_2 \qquad IV$$

in which

E represents a phenyl, imidazolyl, thiazolyl, furyl, thienyl, or pyridyl radical,

which radicals may be unsubstituted or may have one or two substitutents, selected from straight and branched chain alkyl groups having from 1 to 4 carbon atoms, guanidino and mono-alkyl guanidino groups, the alkyl moiety of which has a straight or branched chain and from 1 to 4 carbon atoms, and groups of the formula II

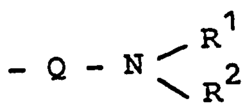

II

in which Q represents an alkylene group having from 1 to 4 carbon atoms, and $R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl substitutent having from 1 to 4 carbon atoms,

X represents -O- or -S-;

n represents 0 or 1; and

m represents 2, 3 or 4,

to give a compound of formula Ia

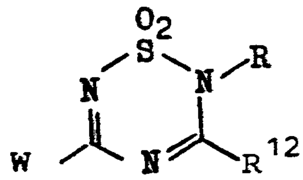

Ia

in which R and $R^{12}$ are as defined above, and W represents a group $E-(CH_2)_n - X -(CH_2)_m - NH-$ in which E, X, n and m are as defined above.

In formula Ia, alkyl groups may be branched or unbranched, and alkyl groups, for example, alkyl groups $R^4$ and $R^5$, may be unsubstituted or substituted, for example, by one or more,
for example, one or two, groups selected from
(i) $-OR^6$ groups in which $R^6$ represents a straight or branched chain alkyl group having from 1 to 6 carbon atoms;
(ii) cycloalkyl groups having from 3 to 7 carbon atoms;
(iii) $-NR^7 R^8$ groups in which $R^7$ and $R^8$, which may be the same or different, each represents a hydrogen atom, a straight or branched chain alkyl group having from 1 to 4 carbon atoms, a phenyl group or an acyl group having from 1 to 4 carbon atoms in the alkyl moiety, and $R^7$ and $R^8$ together with the nitrogen atom to which they are attached may form a 5 or 6 membered ring;
(iv) $-COOR^9$ groups in which $R^9$ represents a straight or branched chain alkyl group having from 1 to 4 carbon atoms;
(v) $-CONR^7 R^8$ groups in which $R^7$ and $R^8$ are defined above;
(vi) alkylsulphonyl groups having from 1 to 4 carbon atoms in the alkyl moiety and phenylsulphonyl groups;
(vii) cyano and nitro groups;
(viii) phenyl groups which may be substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl

moiety having a straight or branched chain and from 1 to 4 carbon atoms;

(ix) aromatic and non-aromatic heterocyclic groups having from 5 to 8 ring members and one or two hetero-atoms selected from oxygen, sulphur and nitrogen atoms, and optionally having an alkyl substitutent having from 1 to 4 carbon atoms on a ring nitrogen atom, for example, a furyl, tetrahydrofuryl, thienyl, pyridyl, dihydropyranyl, pyrrolidinyl, N-lower alkyl-pyrrolidinyl, or piperidyl group.

A phenyl group in formula Ia other than a phenyl group E (which is as defined above), may be unsubstituted or substituted as defined in (viii) above.

Compound XIV is generally reacted with one equivalent of anhydrous ammonia, preferably in an inert solvent, for example, tetrahydrofuran or dioxane, preferably at a temperature in the range of from 0 to 20°C to give compound XV, which is cyclised to the thiatriazine XVII by reaction with ortho ester XVI. This reaction may be carried out using an excess of the ortho ester as solvent or, preferably, using an inert solvent, for example, acetonitrile, tetrahydrofuran, dioxane or diglyme, preferably at an elevated temperature, for example, from 80 to 160°C.

Compound IV is generally reacted with compound XVII in a solvent or diluent, preferably in an alcohol, acetone, acetonitrile or DMF. The reaction temperature is, for example, within the range of from 0 to 80° C, depending on the nature of the leaving group $R^{10}$. Generally when $R^{10}$ is a substituted phenoxy group, the reaction occurs rapidly at 0 to 20°C, but when $R^{10}$ is an alkylthio or alkoxy group, elevated reaction temperatures, for example, in the range of from 50 to 100°C, for example, from 60 to 80°C are preferred.

The compound of formula IV is reacted in the form of the free base, as shown. If it is initially present in the form of an acid addition salt, for example, as the

hydrochloride or hydrobromide, this is converted into the free base during or, preferably, before reaction with compound XVII. Conversion is carried out with an organic or inorganic base, for example, sodium hydroxide or potassium hydroxide, or a tertiary amine, for example, triethylamine.

Compounds of formula XLI and of formula Ia have histamine H-2 antagonist activity, and may be used to treat conditions resulting from stimulation by histamine of H-2 receptors, either alone, for example, in inhibiting gastric acid secretion and thus treating its sequelae, for example, gastric and peptic ulcers; or together with H-1 antagonists, for example, in allergic and certain inflammatory conditions.

Compounds of formula XIV and XLVII are particularly preferred compounds of formula XXXI.

The present invention further provides compounds of formula XXXV

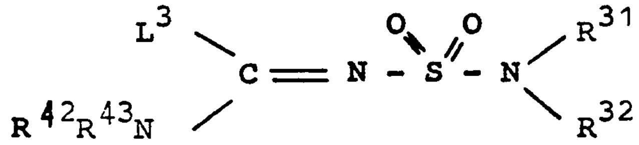

XXXV

in which

$R^{31}$ and $R^{32}$, which may be the same or different, each represents a hydrogen atom, a straight or branched chain alkyl group having from 1 to 4 carbon atoms, a cycloalkyl group having from 3 to 7 carbon atoms, a phenyl group, or a phenalkyl group having from 1 to 4 carbon atoms in the alkyl moiety,

wherein an alkyl, cycloalkyl, phenyl or phenalkyl group may be unsubstituted or substituted by one or more substitutents, which may be the same or different, for example, by one or two substitutents, especially by one substitutent, selected from halogen atoms, straight or branched chain alkyl groups having from 1 to 4 carbon atoms, straight or branched chain alkoxy groups having from 1 to 4 carbon atoms, and nitro and trifluoromethyl groups; or

$R^{31}$ and $R^{32}$ together with the nitrogen atom to which they are attached, form a 4- to 7-membered ring which may contain one or more further hetero-atoms, for example, selected from nitrogen, oxygen and sulphur atoms;

$L^3$ represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms, and

$R^{42}$ and $R^{43}$, which may be the same or different, each represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

wherein an alkyl, phenyl or phenyalkyl group may be unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups.

Compounds of formula XXXV may exist in various isomeric and tautomeric forms. The present invention includes all isomeric and tautomeric forms of a compound of formula XXXV.

In the present specification, the term "lower" denotes groups having up to 4 carbon atoms, and any alkyl group can have a straight or branched chain.

In a compound of formula XXXV, $R^{31}$ preferably represents a hydrogen atom, and $R^{32}$ preferably represents a hydrogen atom or a lower alkyl group, especially a methyl group or an ethyl group. Particularly preferred are those compounds in which $R^{31}$ represents a hydrogen atom and $R^{32}$ represents a methyl group and especially preferred are those compounds in which $R^{31}$ and $R^{32}$ both represent hydrogen atoms.

When X in $L^3$ represents a sulphur atom, $L^3$ preferably represents a lower alkylthio group, especially a methylthio group, or a phenylthio group. When X

represents an oxygen atom, $L^3$ preferably represents a substituted phenoxy group, especially a mono-substituted phenoxy group, for example, a nitro-substituted phenoxy group, for example, an ortho-nitrophenoxy group.

The present invention provides a process for the production of a compound of formula XXXV as defined above, which comprises reacting a compound of formula XXXI

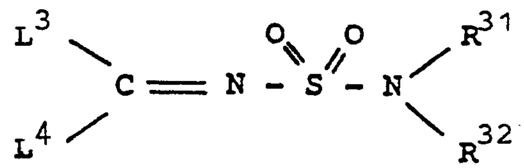

XXXI

in which $R^{31}$ and $R^{32}$ are as defined above, and

$L^3$ and $L^4$, which may be the same or different, each represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms,

with the proviso that in $L^3$ and $L^4$, the two radicals $R^{46}$ may be the same or different but the two radicals X must be the same, with an amine of formula XLVI

$$HNR^{42}R^{43} \qquad XLVI$$

in which $R^{42}$ and $R^{43}$ are as defined above.

The reaction between compounds XXXI and XLVI, respectively, is generally carried out in a solvent, for example, an organic solvent, for example, a lower alcohol, for example, methanol, ethanol or isopropanol, dimethylformamide, tetrahydrofuran or acetonitrile,

or in a mixture of two or more of such solvents. The reaction is generally carried out at a temperature within the range of from 5 to 75°C, especially at room temperature.

The following compounds of formula XXXI are particularly preferred for use as starting materials:

(1) $R^{31}$ = $R^{32}$ = hydrogen, $L^3$ = methylthio and $L^4$ = phenylthio, i.e. S-Methyl,S'-phenyl-N-sulphamoyl-dithioimidocarbonate

(2) $R^{31}$ = hydrogen, $R^{32}$ = methyl, $L^3$ = methylthio and $L^4$ = phenylthio, i.e. S-Methyl,S'-phenyl-N-(N'-methyl)-sulphamoyldithioimidocarbonate;

(3) $R^{31}$ = $R^{32}$ = hydrogen, $L^3$ = $L^4$ = 2-nitrophenyloxy ie Bis-(2-nitrophenyl)-N-sulphamoylimidocarbonate; and

(4) $R^{31}$ = hydrogen, $R^{32}$ = methyl, $L^3$ = $L^4$ = 2-nitrophenyloxy ie Bis-(2-nitrophenyl)-N-(N'-methyl)-sulphamoylimidocarbonate.

A compound of formula XXXI may be prepared by reacting a compound of formula XXXII

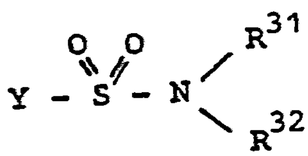 XXXII

in which Y represents a halogen atom, especially a chlorine atom, and $R^{31}$ and $R^{32}$ are as defined above, with a compound of formula XXXIII or with a compound of formula XXXIV

XXXIV 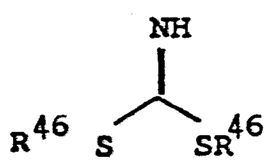 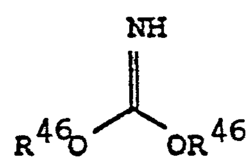 XXXIV

in which $R^{46}$ is as defined above.

A compound of formula XXXII may be prepared as described in Chem. Ber. 91, 1339, (1958), in DE-OS 937,645, J. Org. Chem. 41 4028 (1976) or in J. Med. Chem. 15 (5) 538 (1972), and is preferably used when freshly prepared.

A compound of formula XXXIII may be prepared as described in Z. Chem. 8 (12) 459-460 (1968), Bull. inst. recherches biol unic. Perm, 6, (10), 517-522, or Beilstein E113 page 156, and a compound of formula XXXIV as described in Ann. 287 310, 319 and 321 (1895).

In a compound of formula XXXIII, one radical $R^{46}$ preferably represents a methyl group and the other preferably represents a phenyl group. In a compound of formula XXXIV, both radicals $R^{46}$ preferably represent substituted phenyl groups, for example, ortho-nitrophenyl groups.

The reaction between the compound of formula XXXII and the compound of formula XXXIII or XXXIV is preferably carried out in the presence of an organic base, especially a tertiary amino base, for example, triethylamine.

The reaction is preferably carried out in the presence of an aprotic solvent, for example, acetonitrile, or an ether, an aromatic hydrocarbon, a chlorinated aliphatic or aromatic hydrocarbon, for example, diethyl ether, tetrahydrofuran, benzene or dichloromethane.

The reaction is preferably carried out under an inert atmosphere, for example, under nitrogen. The reaction is generally carried out at a temperature within the range of from -50 to +80°C, peferably from -5 to +45°C.

Preferably, a compound of formula XXXII, advantageously freshly prepared, is added dropwise to a solution of the compound of formula XXXIII or XXXIV, and the base is preferably added simultaneously with the compound of formula XXXII. This addition may be carried out over a period of from 5 minutes to 8 hours, preferably from 10 minutes to 2 hours.

Compounds of formula XXXI in which $R^{31}$ and $R^{32}$ both represent hydrogen atoms and $L^3$ and $L^4$ both represent alkylthio or both represent alkoxy groups are disclosed in UK Patent Specification No. 1 398 426, which discloses a process for the production of those compounds of formula

XXXI in which $R^{31}$ and $R^{32}$ both represent hydrogen atoms and $L^3$ and $L^4$ represent lower alkoxy or lower alkylthio groups.

Compounds of formula XXXV are versatile intermediates which can be used in the production of a wide range of sulphamoyl guanidines, sulphamoyl isothioureas, sulphamoyl thioureas, and a variety of heterocyclic systems by displacement of the leaving group $L^3$ by a suitable group. For example, by reacting a compound of formula XLIII

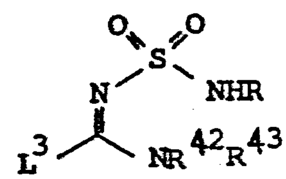

XLIII

in which $L^3$, $R^{42}$ and $R^{43}$ are as defined above and R is as defined above for $R^{42}$ , ($R^{42}$, $R^{43}$ and R being the same or different,) with a compound of formula XLV

$$A - (CH_2)_n - X - (CH_2)_m - NH_2 \qquad \text{XLV}$$

in which A represents a phenyl or furyl radical,

which radical may be unsubstituted or may have one or more, especially one or two, substitutents which may be the same or different, selected from guanidino and mono-alkyl guanidino groups, the alkyl moiety of which has a straight or branched chain and from 1 to 4 carbon atoms, and from groups of formula II

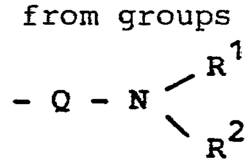

II

in which Q represents an alkylene group having from 1 to 4 carbon atoms, and $R^1$ and $R^2$, which may be the same or different, each represents a hydrogen atom, or a straight or branched chain alkyl group having from 1 to 4 carbon atoms, or $R^1$ and $R^2$ together with the nitrogen atom to which they are attached form a 4 to 8-membered ring which may contain an oxygen atom or another nitrogen atom, which nitrogen atom may have an alkyl group having from 1 to 4 carbon atoms as

substitutent, or

A represents a thiazolyl or thiadiazolyl radical that is substituted by one or more, especially by one or two, substituents which may be the same or different and which are as defined above for substitutents of phenyl and furyl groups A;

X represents an oxygen or sulphur atom;

n represents 0 or 1; and

m represents 2, 3 or 4,

thereis produced a compound of formula XLI

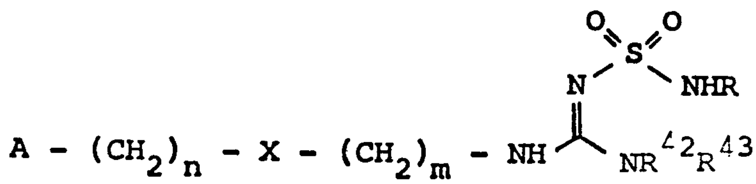

XLI

in which A, X, n, m, R, $R^{42}$ and $R^{43}$ are as defined above.

The reaction between compounds XLIII and XLV is generally carried out in a solvent, for example, an organic solvent, for example, a lower alcohol, for example, methanol, ethanol or isopropanol, dimethylformamide, tetrahydrofuran or acetonitrile, or in a mixture of two or more of such solvents. The reaction is generally carried out at a temperature within the range of from 5 to 75°C, especially at room temperature.

Compounds of formula XLI and processes for their production are described and claimed in our co-pending application no. (Case 19). Compounds analogous to compounds XLI may be prepared analogously from other compounds of formula XXXV.

An example of the use of a compound of formula XXXV in the production of a heterocyclic system is as follows:

A compound of formula XV

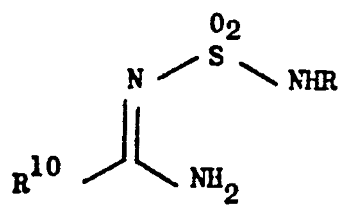

XV

in which R is as defined above, and

$R^{10}$ represents an alkoxy, phenoxy, phenylalkoxy,

alkylthio, phenylthio or phenylalkylthio group wherein a phenyl or phenalkyl group may be unsubstituted or substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl moiety having a straight or branched chain and from 1 to 4 carbon atoms,
is reacted with a compound of formula XVI

$$R^{12} - C(OR^3)_3 \qquad XVI$$

in which $R^3$ represents a straight or branched chain alkyl group having from 1 to 4 carbon atoms, and $R^{12}$ represents a hydrogen atom, an alkyl group having from 1 to 6 carbon atoms, or an $-OR^3$ group, in which $R^3$ is as defined above, to give a compound of formula XVII

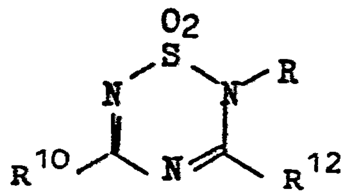

XVII

in which R, $R^{10}$ and $R^{12}$ are as defined above.

A compound of formula XVII may then be reacted with a compound of formula IV

$$E - (CH_2)_n - X - (CH_2)_m - NH_2 \qquad IV$$

in which E represents a phenyl, imidazolyl, thiazolyl, furyl, thienyl, or pyridyl radical,
which radicals may be unsubstituted or may have one or two substitutents, selected from straight and branched chain alkyl groups having from 1 to 4 carbon atoms, guanidino and mono-alkyl guanidino groups, the alkyl moiety of which has a straight or branched chain and from 1 to 4 carbon atoms, and groups of the formula II

$$- Q - N\begin{matrix} R^1 \\ R^2 \end{matrix} \qquad II$$

in which Q represents an alkylene group having from 1 to 4 carbon atoms, and $R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl substituent having from 1 to 4 carbon atoms,

X represents -O- or -S-;

n represents 0 or 1; and

m represents 2, 3 or 4,

to give a compound of formula Ia

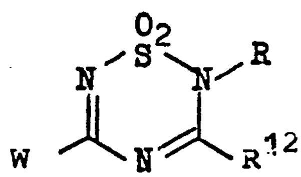

Ia

in which R and $R^{12}$ are as defined above, and W represents a group $E-(CH_2)_n - X -(CH_2)_m - NH-$ in which E, X, n and m are as defined above.

In formula Ia, alkyl groups may be branched or unbranched, and alkyl groups, for example, alkyl groups $R^4$ and $R^5$, may be unsubstituted or substituted, for example, by one or more,

for example, one or two, groups selected from

(i) $-OR^6$ groups in which $R^6$ represents a straight or branched chain alkyl group having from 1 to 6 carbon atoms;

(ii) cycloalkyl groups having from 3 to 7 carbon atoms;

(iii) $-NR^7R^8$ groups in which $R^7$ and $R^8$, which may be the same or different, each represents a hydrogen atom, a straight or branched chain alkyl group having from 1 to 4 carbon atoms, a phenyl group or an acyl group having from 1 to 4 carbon atoms in the alkyl moiety, and $R^7$ and $R^8$ together with the nitrogen atom to which they are attached may form a 5 or 6 membered ring;

(iv) $-COOR^9$ groups in which $R^9$ represents a straight or branched chain alkyl group having from 1 to 4 carbon atoms;

(v) $-CONR^7 R^8$ groups in which $R^7$ and $R^8$ are defined above;

(vi) alkylsulphonyl groups having from 1 to 4 carbon atoms in the alkyl moiety and phenylsulphonyl groups;

(vii) cyano and nitro groups;

(viii) phenyl groups which may be substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl moiety having a straight or branched chain and from 1 to 4 carbon atoms;

(ix) aromatic and non-aromatic heterocyclic groups having from 5 to 8 ring members and one or two hetero-atoms selected from oxygen, sulphur and nitrogen atoms, and optionally having an alkyl substitutent having from 1 to 4 carbon atoms on a ring nitrogen atom, for example, a furyl, tetrahydrofuryl, thienyl, pyridyl, dihydropyranyl, pyrrolidinyl, N-lower alkyl-pyrrolidinyl, or piperidyl group.

A phenyl group in formula Ia other than a phenyl group E (which is as defined above), may be unsubstituted or substituted as defined in (viii) above.

Compound XV is cyclised to the thiatriazine XVII by reaction with ortho ester XVI. This reaction may be carried out using an excess of the ortho ester as solvent or, preferably, using an inert solvent, for example, acetonitrile, tetrahydrofuran, dioxane or diglyme, preferably at an elevated temperature, for example, from 80 to 160°C.

Compound IV is generally reacted with compound XVII in a solvent or diluent, preferably in an alcohol, acetone, acetonitrile or DMF. The reaction temperature is, for example, within the range of from 0 to $80^\circ$ C,

depending on the nature of the leaving group $R^{10}$. Generally when $R^{10}$ is a substituted phenoxy group, the reaction occurs rapidly at 0 to 20°C, but when $R^{10}$ is an alkylthio or alkoxy group, elevated reaction temperatures, for example, in the range of from 50 to 100°C, for example, from 60 to 80°C are preferred.

The compound of formula IV is reacted in the form of the free base, as shown. If it is initially present in the form of an acid addition salt, for example, as the hydrochloride or hydrobromide, this is converted into the free base during or, preferably, before reaction with compound XVII. Conversion is carried out with an organic or inorganic base, for example, sodium hydroxide or potassium hydroxide, or a tertiary amine, for example, triethylamine.

Compounds of formula XLI and of formula Ia have histamine H-2 antagonist activity, and may be used to treat conditions resulting from stimulation by histamine of H-2 receptors, either alone, for example, in inhibiting gastric acid secretion and thus treating its sequelae, for example, gastric and peptic ulcers; or together with H-1 antagonists, for example, in allergic and certain inflammatory conditions.

Compounds of formula XV and XLIII are particularly preferred compounds of formula XXXV.

The following Examples illustrate the invention, but are not limiting. In the Examples, ratios of eluants are expressed by volume.

EXAMPLE A

1. 3-[2-[(2-Guanidino-4-thiazolyl)methylthio]ethyl]amino-5-methylamino-6-methyl-1,2,4,6-thiatriazine-1,1,-dioxide

To 604mg of 2-guanidino-4-[(2-aminoethyl)thiomethyl]-thiazole dihydrochloride was added a solution of 224mg of potassium hydroxide dissolved in 5ml of anhydrous ethanol. The resulting suspension was stirred for a few minutes and then filtered to give a clear ethanolic solution of the guanidino-thiazole free base, which was used for the following reactions.

a) To an ethanolic solution of the free base prepared as described above was added 444mg of 3-methylthio-5-methylamino-6-methyl-1,2,4,6-thiatriazine-1,1-dioxide dissolved in 10ml of dry acetonitrile. The reaction mixture was heated to reflux for 5 days, after which tlc analysis indicated approximately 30% reaction. The reaction solvents were evaporated and the residual oil chromatographed on silica gel, using as eluant chloroform: methanol:concentrated aqueous ammonia (90:10:1). 280mg of unchanged thiatriazine was recovered from this chromatography, followed by 120mg of purified title compound, obtained as a foam after drying *in vacuo*.

NMR (60MHz) $CD_3OD$ =

2.72 (2H, m), 2.92 (3H, d), 3.26 (3H, s),
3.32 (2H, m), 3.70 (2H, s), 6.65 (1H, d)

b) To 5ml of an ethanolic solution of 0.5mmole of the guanidinothiazole free base was added 273mg of a crude preparation of 3-methylsulphinyl-5-methylamino-6-methyl-1,2,4,6-thiatriazine-1,1-dioxide dissolved in 5ml of dry acetonitrile. After having been stirred at room temperature for 20 minutes, the reaction mixture showed no starting material by tlc analysis. The reaction solvents were

evaporated off, and the residual oil chromatographed on silica gel as described above to give 112mg of the title compound as a semi-crystalline solid. The hydrochloride salt was formed with anhydrous HCl in ethanol, and recrystallised from ethanol/acetonitrile, m.p. 156-165°C.

MNR (60MHz) DMSO-$d_6$ of free base =
2.54 (2H, m), 2.86 (3H, d), 3.15 (3H, s),
3.34 (2H, m), 3.66 (2H, s), 6.68 (1H, d),
7.30 (4H, br.s).

c) To 10ml of an ethanolic solution of 2mmoles of the guanidinothiazole free base prepared as described above was added 200mg of triethylamine followed by 420mg of 3-chloro-5-methylamino-6-methyl-1,2,4,6-thiatriazine-1,1-dioxide dissolved in 5ml of dry acetonitrile. After having been stirred at room temperature for 15 minutes, the reaction mixture showed no starting material by tlc analysis. The title compound was obtained as a semi-crystalline solid by the chromatographic procedure described above. Treatment with anhydrous HCl as described above gave the pure HCl salt, which was crystallised from ethanol/acetonitrile: 632mg, m.p. 159-165°C.

NMR (60MHz) DMSO-$d_6$ of HCl salt =
2.54 - 2.66 (2H, m), 2.79 (3H, d), 3.15 (3H, s),
3.25 - 3.42 (2H, m), 3.77 (2H, s), 7.15 (1H, d),
7.60 - 7.86 (2H, m), 8.34 (4H, br.s)

2. <u>3-[2-[(2-Guanidino -4-thiazolyl)methylthio]ethyl]amino-5-tert.-butylamino-6-methyl-1,2,4,6-thiatriazine-1,1-dioxide</u>

To 10ml of an ethanolic solution of 1.5mmole of 2-guanidino -4-[(2-aminoethyl)thiomethyl]thiazole free base prepared as described in Example A1 above, was added 380mg of 3-chloro-5-*tert.*-butylamino-6-methyl-1,2,4,6-thiatriazine-1,1-dioxide dissolved in 5ml of dry acetonitrile. After having been stirred for 2 hours, the reaction mixture was evaporated to dryness and the residue was chromatographed on silica gel using as eluant chloroform/methanol/concentrated

aqueous ammonia (90:10:1). 620mg of the title compound was obtained as a foam after drying _in vacuo_.

NMR (250MHz) DMSO-$d_6$ =
1.39 (9H, s), 2.57 (2H, m), 3.19 (3H, d),
3.23 - 3.44 (2H, m), 3.60 (2H, s), 6.48 - 6.55 (1H, m),
6.90 (5H, br.s), 7.61 - 7.76 (1H, 2xt)

3. <u>3-[3-[3-(1-Piperidinylmethyl)phenoxy]propylamino-5-tert.-butylamino-6-methyl-1,2,4,6-thiatriazine-1,1,-dioxide</u>

496mg of 3-[3-(1-piperidinylmethyl)phenoxy]propylamine were dissolved in 10ml of dry acetonitrile. 504mg of 3-chloro-5-_tert._-butylamino-6-methyl-1,2,4,6-thiatriazine-1,1-dioxide dissolved in 10ml of dry acetonitrile were added dropwise at room temperature to the stirred solution of the amine. After stirring for 1 hour, the solvent was evaporated and the residual oil chromatographed on silica gel using $CHCl_3$/MeOH/conc. aqueous ammonia (90:10:1). After drying under vacuum, 628mg of the title compound was isolated as a white foam.

NMR (250MHz) DMSO-$d_6$ =
1.41 (9H, d), 1.46 (6H, m), 1.89 (2H, m),
2.28 (4H, m), 3.18 (3H, d), 3.35 (2H, s),
3.25 (2H, m), 3.97 (2H, m), 6.37 (1H, d),
6.76 - 6.84 (3H, m), 7.12 (1H, t),
7.59 - 7.67 (1H, 2xt)

<u>EXAMPLE B</u>

1. <u>3-Chloro-5-methylamino-6-methyl-1,2,4,6-thiatriazine-1,1-dioxide</u>

666mg of 3-methylthio-5-methylamino-6-methyl-1,2,4,6-thiatriazine-1,1-dioxide were suspended in 20ml of dry methylene dichloride. Chlorine gas was bubbled slowly into the stirred suspension at room temperature until the yellow colour of excess chlorine persisted in the reaction mixture. After having been stirred for 30 minutes, the now clear reaction solution was evaporated to dryness, leaving a white

crystalline solid which was recrystallised from acetone/hexane to yield the title compound: 573mg, m.p. 179-180°C.

NMR (60MHz) in $CD_3CN$: = 2.95 (3H, d), 3.30 (3H, s)

2. 3-Chloro-5-tert.-butylamino-6-methyl-1,2,4,6-thiatriazine-1,1-dioxide

400mg of 3-methylthio-5-tert.-butylamino-6-methyl-1,2,4,6-thiatriazine-1,1-dioxide were reacted with excess chlorine in 15ml of dry methylene dichloride as described above for example B1. 319mg of the title compound were obtained, m.p. 143-145°C.

NMR (60MHz) acetone-$d_6$: = 1.48(9H, s), 3.39 (3H, s)

## EXAMPLE C

3-Methylsulphinyl-5-methylamino-6-methyl-1,2,4,6-thiatriazine-1,1-dioxide

222mg of 3-methylthio-5-methylamino-6-methyl-1,2,4,6-thiatriazine-1,1-dioxide were suspended in 5ml of dry chloroform and 410mg of m-chloroperoxybenzoic acid (approx. 80% purity) dissolved in 5ml of methylene dichloride added dropwise to this suspension. A clear solution formed quickly, but after about 30 minutes, a crystalline precipitate began to form. After stirring for 2 hours at room temperature, the thick white precipitate was filtered off and washed thoroughly with ether. After drying, 273mg of crude sulphinylthiatriazine was obtained. This product was sufficiently pure for further reaction. Tlc analysis indicated the presence of some m-chlorobenzoic acid.

NMR (60MHz) DMSO-$d_6$: =
2.85 (3H, d), 3.00 (3H, s), 3.30 (3H, s)

## EXAMPLE D

1. 3-Methylthio-5-methylamino-6-methyl-1,2,4,6-thiatriazine-1,1-dioxide

3.34g of 1-methyl-2-S-benzyl-4-S-methyldi-isothiobiuret hydrobromide was dissolved in 50ml of methylene dichloride

containing 1.10g of triethylamine. 1.42g of methylaminosulphonyl chloride and another 1.10g of triethylamine were added dropwise simultaneously to the stirred solution of the di-isothiobiuret. After having been stirred for 2 hourss, the reaction mixture was evaporated to a semi-crystalline solid, which was suspended in 50ml of dry acetonitrile and filtered to remove the crystalline triethylamine hydrochloride. The filtrate was heated to reflux for 1 hour, then evaporated to dryness and the residue chromatographed on silica gel using $CHCl_3$/MeOH (9:1) to yield the title compound, which was crystallised from acetone/hexane, m.p. 189-190°C.

NMR (60MHz) acetone-$d_6$: =

2.42 (3H, s), 3.02 (3H, d), 3.35 (3H, s)

Mass spectrum: 222(m/e), 158, 143, 71.

2. <u>3-Methylthio-5-tert.-butylamino-6-methyl-1,2,4,6-thiatriazine-1,1-dioxide</u>

1.4g of 1-tert.-butyl-2-S-benzyl-4-S-methyl-di-isothiobiuret hydrobromide was reacted with 0.6g of methylaminosulphonyl chloride as described for Example D1 to give 730mg of the title compound.

NMR (60MHz) acetone-$d_6$ =

1.50 (9H, s), 2.36 (3H, s), 3.30 (3H, s)

EXAMPLE E

1. 1-Methyl-2-S-benzyl-4-S-methyldi-isothiobiuret hydrobromide salt.

13.9g of methyl isothiourea sulphate was suspended in 500ml of methylene dichloride. With vigorous stirring, 4.40g of sodium hydroxide dissolved in 50ml of water was added, followed immediately by 7.30g of methyl isothiocyanate. After having been stirred at room temperature for 2 hours, the reaction mixture was filtered, and the filtrate evaporated to dryness. The residual oil was chromatographed on silica gel with $CHCl_3$/MeOH (9:1) to give 7.70g of 1-methyl-4-S-methyldithiobiuret as a colourless oil.

NMR (60MHz) $CDCl_3$: = 2.36 (3H, s), 3.30 (3H, d)

3.26g of this oil was refluxed for 1 hour in 50 ml of acetonitrile with 3.42g of benzyl bromide. The solvent was then concentrated to about 10ml and ether added to start crystallisation of the product. This was filtered off, washed with ether and dried to give 3.90g of the title compound, m.p. 151-152°C.

NMR (60MHz) DDMSO-$d_6$: =

2.38 (3H, s), 3.04 (3H, s), 4.36 (2H, s),
7.26 (5H, s), 8.70 (2H, br.s)

2. <u>1-Tert.-butyl-2-S-benzyl-4-S-methyldi-isothiobiuret hydrobromide salt</u>

2.78g of methyl isothiourea sulphate in 50ml of methylene dichloride was reacted with 880mg of sodium hydroxide dissolved in 5ml of water and 3.45g of <u>t</u>-butylisothiocyanate as described in Example E1, except that after having been stirred for 2 hours at room temperature, the reaction mixture was refluxed for 1 hour. Chromatography gave 507mg of 1-<u>tert.</u>-butyl-4-S-methyldithiobiuret, which was then crystallised from hexane, m.p. 132-133°C.

NMR (60MHz) $CDCl_3$: = 1.41 (9H, s), 2.46 (3H, s)

Reaction of 1-<u>tert.</u>-butyl-4-S-methyldithiobiuret with benzyl bromide as described in Example E1 gave 830mg of the title compound, m.p. 152-156°C.

NMR (60MHz) DMSO-$d_6$: =

1.32 (9H, s), 2.33 (3H, s), 4.18 (2H, s),
7.28 (5H, s), 9.28 (2H, br.s)

<u>EXAMPLE F</u>

<u>3-[2-[(2-Guanidino-4-thiazolyl)methylthio]ethyl]amino-5-methyl-6-methyl-1,2,4,6-thiatriazine-1,1-dioxide</u>

302mg of 2-guanidino-4-[(2-aminoethyl)thiomethyl]thiazole dihydrochloride were treated with 112mg of potassium hydroxide dissolved in 5ml of ethanol as described for

Example A1. To the ethanol solution of the guanidinothiazole free base was added 298mg of 3-(2-nitrophenoxy)-5-methyl-6-methyl-1,2,4,6-thiatriazine-1,1-dioxide dissolved in 10ml of tetrahydrofuran. The reaction mixture was stirred at room temperature for 18 hours, then evaporated to dryness, and the residual oil chromatographed on silica gel with $CHCl_3$/MeOH/conc. aqueous ammonia (90:10:1). The product was isolated, and crystallised from acetone/ethanol to give 326mg of the title compound, m.p. 171-172°C.

NMR (250MHz) DMSO-$d_6$: =

2.31 (3H, s), 2.58 (2H, t), 3.36 (3H, s),
3.22 - 3.49 (2H, m), 3.60 (2H, s), 6.52 (1H, s),
6.90 (4H, br.s), 8.17 - 8.53 (1H, 2xt)

EXAMPLE G

3-(2-Nitrophenoxy)-5-methyl-6-methyl-1,2,4,6-thiatriazine-1,1-dioxide

1.37g of 1-(N'-methyl)sulphamoyl-2-(2-nitrophenyl)-isourea and 2.42g of trimethyl orthoacetate were refluxed for 3 hours in 10ml of acetonitrile. The solvent was evaporated and the residual solid washed with several portions of ether before recrystallisation from acetone to give the title compound: 810mg, m.p. 212-214°C.

NMR (250MHz) $CDCl_3$: =

2.49 (3H, s), 3.57 (3H, s), 7.35 (1H, d),
7.48 (1H, t), 7.71 (1H, t), 8.17 (1H, d)

EXAMPLE H

1-(N'-methyl)sulphamoyl-2-(2-nitrophenyl)-isourea

3.96g of bis-(2-nitrophenyl)-N-(N'-methyl)-sulphamoyl imidocarbonate was suspended in 50 ml of tetrahydrofuran, and 170mg of anhydrous ammonia dissolved in 5ml of tetrahydrofuran was added to the cooled (+5°C), rapidly stirred suspension. The resulting clear solution was allowed to come to ambient temperature over 1 hour, when tlc analysis (silica gel, $CHCl_3$/MeOH, 5:1), indicated the

absence of starting material. The solvent was evaporated off and the residual solid washed with ether and finally recrystallised from acetone/ether to give the title compound: 2.32g, m.p. 150-151°C

NMR (250MHz) DMSO-$d_6$: =
2.32 (3H, d), 6.67 (1H, m), 7.40 (1H, br.s),
7.51 (2H, m), 7.82 (1H, t), 8.14 (1H, d),
8.81 (1H, br.s)

EXAMPLE I

Bis-(2-nitrophenyl)-N-(N'-methyl)sulphamoylimido carbonate

6.06g of bis-(2-nitrophenyl)imido carbonate was dissolved in 150ml of dry methylene dichloride. 2.84g of methylsulphamoyl chloride and 2.20g of triethylamine were added dropwise simultaneously to this solution with rapid stirring. After having been stirred for 15 minutes, the reaction solution was washed twice with water, dried over $MgSO_4$, and evaporated to a crystalline residue which was washed with ether and recrystallised from acetone/ether to give the title compound: 6.50g, m.p. 171-172°C.

NMR (60MHz) DMSO-$d_6$: = 2.50 (3H, d), 7.1 - 8.3 (8H, m)

EXAMPLE J

1. 2-Methyl-3-[2-[(2-guanidino-4-thiazolyl)methylthio]-ethyl]amino-5-tert.-butylamino-1,2,4,6-thiatriazine-1,1-dioxide

302mg of 2-guanidino-4-[(2-aminoethyl)thiomethyl]thiazole dihydrochloride was suspended in 10ml of ethanol containing 112mg of potassium hydroxide. The suspension was stirred for a few minutes and then filtered to give a clear ethanol solution of the guanidino thiazole free base. To this solution was added 110mg of triethylamine followed by 252 mg of 2-methyl-3-chloro-5-t-butylamino-1,2,4,6-thiatriazine-1,1-dioxide dissolved in 2ml of dry acetonitrile. The reaction solution was stirred for 1 hour at room temperature, then evaporated to dryness, and the residual oil chromatographed on silica gel with $CHCl_3$/MeOH. The

crude product (535mg) was recrystallised twice from ethanol to give the pure title compound: 433mg, m.p. 110-112°C.

NMR (400MHz) DMSO-$d_6$: =

1.33 (9H, s), 2.67 (2H, m), 3.14(3H, d), 3.25 - 3.55 (2H, m), 3.64 (2H, s), 6.52 (1H, d), 6.89 (4H, br.s), 7.04 (½H, s), 7.18 (½H, s), 7.59 (½H, t), 7.84 (½H, t).

2. <u>2-Methyl-3-[3-[3-(1-piperidinylmethyl)phenoxy]propyl]-amino-5-t-butylamino-1,2,4,6-thiatriazine-1,2-dioxide.HCl salt</u>

143mg of 2-methyl-3-chloro-5-<u>t</u>-butylamino-1,2,4,6-thiatriazine-1,1-dioxide was dissolved in 5ml of dry acetonitrile and added dropwise to a stirred solution of 140mg of 3-[3-(1-piperidinylmethyl)phenoxy]propylamine in 5ml of dry acetonitrile. After having been stirred at room temperature for 2 hours, the reaction solution was evaporated to dryness and the residual oil was chromatographed on silica gel with $CHCl_3$/MeOH (4:1). The title compound was obtained as a foam after drying <u>in vacuo</u>: 163mg

NMR (250 MHz) $CDCl_3$: =

1.41 (9H, s), 1.57 (2H, m), 1.79 (4H, m) 2.13 (2H, m), 2.71 (4H, br.m), 3.37 (3H, d), 3.54 - 3.76 (4H, m)< 4.17 (2H, m), 6.90 (2H, m), 7.27 (2H, m)

<u>EXAMPLE K</u>

1. <u>3-Chloro-5-tert.-butylamino-6-methyl-1,2,4,6-thiatriazine-1,1-dioxide</u>

1.29g of 3,5-dichloro-6-methyl-12,4,6,-thiatriazine-1,1-dioxide was dissolved in 70 ml of anhydrous ether (dried over sodium), and to this well stirred solution at room temperature was added dropwise 876mg of <u>tert.</u>-butyl- amine in 5ml of ether. After having been stirred for 20 minutes, the reaction mixture was filtered and the filtrate reduced in volume to about 10 ml to start crystallisation of the

title compound. 575mg of crude product was filtered off and recrystallised from ether to give 493mg of purified material, m.p. 145-147°C.
NMR (250MHz) $CDCl_3$: = 1.52 (9H, s), 3.36 (3H, s)

2. 2-Methyl-3-chloro-5-tert.-butylamino-1,2,4,6-thiatriazine-1,1-dioxide

The ether filtrate from the fractional crystallisation of the compound of Example K1 was chromatographed on silica gel with $CH_2Cl_2$ to give the title compound, which was recrystallised from ether/hexane: 311mg, m.p. 153-154°C.
NMR (250MHz) $CDCl_3$: = 1.44 (9H, s), 3.38 (3H, s).

EXAMPLE L

3,5-Dichloro-6-methyl-1,2,4,6-thiatriazine-1,1,-dioxide

2.0g of 3-methylthio-6-methyl-1,2,4,6-thiatriazin-5-one-1,1-dioxide were refluxed for 20 hours in 20ml of $POCl_3$ with 8.0g of $PCl_5$. The resulting clear solution was evaporated under vacuum and the residue triturated with dry methylene dichloride. The methylene dichloride extract was filtered through a silica gel column and the product eluted with additional methylene dichloride. The title compound was obtained after recrystallisation from ether/hexane: 1.5g, m.p. 79-80°C.
NMR (60MHz) $CDCl_3$: = 3.70 (3H, s).
Mass Spec. 219 (m+4), 217(m+2), 215(m/e), 180.

EXAMPLE M

1. 2-Methyl-3-[2-[(2-guanidino-4-thiazolyl)methylthio]-ethyl]amino-5-dimethylamino-1,2,4,6-thiatriazine-1,1-dioxide

604mg of 2-guanidino-4-[(2-aminoethyl)thiomethyl]thiazole dihydrochloride was converted into its free base as described in Example A1. 202mg of triethylamine was added to the ethanol solution followed by 492mg of 2-methyl-3-chloro-5-dimethylamino-1,2,4,6-thiatriazine-1,1-dioxide added dropwise in 10ml of dry acetonitrile. After having

been stirred for 30 minutes at room temperature, the reaction solution was evaporated to dryness and the residual oil chromatographed on silica gel with $CHCl_3$/MeOH/conc. aqueous ammonia (50:10:1). After drying in vacuo the title compound was isolated as a foam: 601mg.

NMR (60MHz) DMSO-$d_6$: =

2.78 (2H, t), 3.05 (6H, d), 3.23 (3H, s),
3.65 (2H, t), 3.74 (2H, s), 6.76 (1H, s).

2. 2-Methyl-3-[2-[(2-guanidino-4-thiazolyl)methylthio]-ethyl]amino-5-methoxy-1,2,4,6-thiatriazine- 1,1-dioxide

604mg of 2-guanidino-4[(2-aminoethyl)thiomethyl]thiazole dihydrochloride and 422mg of 2-methyl-3-chloro-5-methoxy-1,2,4,6-thiatriazine-1,1-dioxide were reacted according to the procedure of Example M1 to give the title compound. After chromatography and drying in vacuo, 540mg of the title compound were obtained as a foam.

NMR (250MHz) DMSO-$d_6$: =

2.68 (2H, t), 3.26 (3H, s), 3.32 - 3.55 (2H, m),
3.74 (2H, s), 3.77 (3H, s), 6.96 (1H, s),
7.85 (4H, br. s), 8.59 (1H, t).

3. 2-Methyl-3-[2-[(2-guanidino-4-thiazolyl)methylthio]-ethyl]amino-5-phenyl-1,2,4,6-thiatriazine-1,1-dioxide

302mg of 2-guanidino-4-[(2-aminoethyl)thio-methyl]thiazole dihydrochloride and 257mg of 2-methyl-3-chloro-5-phenyl-1,2,4,6-thiatriazine-1,1-dioxide were reacted according to the procedure of Example M1 to give the title compound. After chromatography, the material was recrystallised from ethanol to give 403mg of the title compound, m.p. 139-143°C.

NMR (250MHz) DMSO-$d_6$: =

2.78 (2H, t), 3.33 (3H, s), 3.68 (2H, s),
3.74 (2H, m), 6.50 (1H, s), 6.92 (4H, br.s)
7.48 - 7.65 (3H, m), 8.17 - 8.21 (2H, d), 8.57 (1H, t)

The chlorothiatriazines used in Examples M1, M2 and M3 were prepared by known methods see, for example, DT-OS 2,943,703 and DT-OS 3,134,143.

EXAMPLE N

1. 3-[2-[(2-Guanidino-4-thiazolyl)methylthio]ethyl]amino-5-amino-6-methyl-1,2,4,6-thiatriazine-1,1-dioxide trifluoroacetic acid salt

300mg of 3-[2-[(2-guanidino-4-thiazolyl)methylthio]ethyl]-amino-5-*tert.*-butylamino-6-methyl-1,2,4,6-thiatriazine-1,1-dioxide was dissolved in 5ml of trifluoroacetic acid and heated at reflux for 5 hours. The trifluoroacetic acid was then evaporated off and the residue recrystallised from ethanol/ether: 236mg, m.p. 110-114°C.

NMR (250MHz) DMSO-$d_6$: =
2.54 (2H, t), 3.12 (3H, s), 3.29 (2H, m)
3.77 (2H, s), 7.13 (1H, d), 7.47 (1H, t),
8.41 (4H, br.s).

2. 2-Methyl-3-[2-[(2-guanidino-4-thiazolyl)methylthio]-ethyl]amino-5-amino-1,2,4,6-thiatriazine-1,1-dioxide trifluoroacetic acid salt

100mg of 2-methyl-3-[2-[(2-guanidino-4-thiazolyl)methylthio]ethyl]amino-5-tert.-butylamino-1,2,4,6-thiatriazine-1,1-dioxide was refluxed in 8ml of trifluoroacetic acid for 18 hours. Evaporation of the trifluoroacetic acid and recrystallisation of the residue from acetone/ether gave the title compound: 84mg. m.p. 135-138°C.

NMR (200MHz) DMSO-$d_6$: =
2.65 (2H, t), 3.14 (3H, s), 3.24 - 3.46 (2H, m),
3.79 (2H, s), 7.09 (1H, s), 7.76 (1H, t),
8.35 (4H, br.s).

3. 3-[3-[3-(1-Piperidinylmethyl)phenoxy]propyl]amino-5-amino-6-methyl-1,2,4,6-thiatriazine-1,1-dioxide trifluoroacetic acid salt

400mg of 3-[3-[3-(1-piperidinylmethyl)phenoxy]propyl]amino-5-*tert.*-butylamino-6-methyl-1,2,4,6-thiatriazine-1,1-dioxide was refluxed in 20ml of trifluoroacetic acid for 4 hours. Evaporation and drying under vacuum gave the title compound as a foam: 396mg.

NMR (250MHz) DMSO-$d_6$: =
1.64 - 1.80 (6H, m), 1.93 (2H, t), 2.86 (2H, m), 3.15 (3H, s), 3.17 - 3.34 (4H, m), 4.02 (2H, m), 4.32 (2H, d), 7.05 (2H, m), 7.34 - 7.49 (3H, m), 7.60 (2H, br.s), 9.32 (1H, br.s).

4. <u>2-Methyl-3-[3-[3-(1-piperidinylmethyl)phenoxy]propyl]-amino-5-amino-1,2,4,6-thiatriazine-1,1-dioxide trifluoroacetic acid salt</u>

150mg of 2-methyl-3-[3-[3-(1-piperidinylmethyl)phenoxy]-propyl]amino-5-*tert.*-butylamino-1,2,4,6-thiatriazine-1,1-dioxide hydrochloride was refluxed for 18 hours in 10ml of trifluoroacetic acid. The trifluoroacetic acid was then evaporated under vacuum and the residual oil crystallised and recrystallised from ethanol: 120mg, m.p. 189-194°C.
NMR (250MHz) DMSO-$d_6$: =
1.54 - 1.84 (6H, m), 2.01 (2H, t), 2.87 (2H, m), 3.15 (3H, s), 3.28 - 3.49 (4H, m), 4.04 (2H, t), 4.22 (2H, d), 7.02 (2H, br.s), 7.07 (3H, m), 7.38 (1H, t), 7.65 (1H, t), 9.39 (1H, br.s).

## EXAMPLE O

### Bis-methyl-N-sulphamoyldithioimidocarbonate

To a solution of 3.75g of dithioimidocarbonate dimethyl ester in 100ml of dry benzene, 4.35 ul of triethylamine and a solution of 3.58g of freshly prepared sulphamoyl chloride in 40ml of dry benzene were added dropwise simultaneously at 18°C, with vigorous stirring under an atmosphere of nitrogen. The addition was carried out over 30 minutes. After a further period of one hour at room temperature, the mixture was evaporated to dryness and triturated with chloroform. The resulting solid was filtered off and dried over silica gel: 3.37g m.p. 148-150°C. m/e 200.

## EXAMPLE P

### Methyl,phenyl-N-sulphamoyl dithioimidocarbonate

To a solution of 8.69g of methyl,phenyl-dithioimidocarbonate in 175ml of dry benzene, 8.14ml of triethylamine and a solution of 6.67g of freshly prepared sulphamoyl chloride in 80ml of dry benzene were added dropwise simultaneously at 18°C with vigorous stirring under an atmosphere of nitrogen. The addition was carried out over a period of one hour. External cooling was applied to maintain the temperature of 18°C. After a further 16 hours at room temperature, 100ml of water and 100ml of diethyl ether were added and the mixture was stirred until all the solids had dissolved. The layers were then separated and the organic phase washed with water, dried over $MgSO_4$, and then evaporated to give an oily solid which on trituration with benzene/hexane gave 4.5g of a colourless solid.

Crystallisation from chloroform gave needles, m.p. 125-126°C. m/e 262

$^1$H nmr 60MHz $CDCl_3$ : 2.40 (3H, s); 5.35 (2H, s. exch); 7.57 (5H, m).

EXAMPLE Q

Bis-(2-nitrophenyl)-N-sulphamoylimidocarbonate

To a vigorously stirred solution of 15.0g of bis-(2-nitrophenyl)-imidocarbonate in a mixture of 200ml of dry benzene, 100ml of ether and 25ml of acetonitrile at 40°C, 7.6ml of triethylamine and a solution of 5.7g of freshly prepared sulphamoyl chloride in 70ml of dry benzene were added dropwise simutaneously over a period of 45 minutes. After one hour at room temperature, 100ml of water and 100ml of ethyl acetate were added to dissolve any solids present. The layers were then separated and the aqueous layer extracted with a further 100ml of ethyl acetate. The combined organic layers were washed with water, dried over $MgSO_4$, and then evaporated to give an oily solid, which on triturationwith ether gave 6.0g of a pale yellow solid, m.p. 165-167°C.

$^1$H nmr 60MHz DMSO-$d_6$ : 4.35 (2H, hump, exch); 7.1-8.3 (8H, m).

EXAMPLE R

Bis-(2-nitrophenyl)-N-(N'-methyl)sulphamoylimido carbonate

6.06g of bis-(2-nitrophenyl)imido carbonate was dissolved in 150ml of dry methylene dichloride. 2.84g of methylsulphamoyl chloride and 2.20g of triethylamine were added dropwise simultaneously to this solution with rapid stirring. After having been stirred for 15 minutes, the reaction solution was washed twice with water, dried over $MgSO_4$, and evaporated to a crystalline residue which was washed with ether and recrystallised from acetone/ether to give the title compound: 6.50g, m.p. 171-172°C.

$^1H$ nmr 60MHz DMSO-$d_6$: 2.50 (3H, d), 7.1 - 8.3 (8H, m).

CLAIMS:

1. A compound of formula I

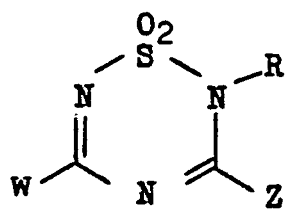

I

in which formula

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

W and Z, which may be the same or different, each represents a group $E-(CH_2)_n - X -(CH_2)_m - NH-$ in which E represents a phenyl, imidazolyl, thiazolyl, furyl, thienyl, or pyridyl radical,

which radicals may be unsubstituted or may have one or two substitutents, selected from straight and branched chain alkyl groups having from 1 to 4 carbon atoms, guanidino and mono-alkyl guanidino groups, the alkyl moiety of which has a straight or branched chain and from 1 to 4 carbon atoms, and groups of the formula II

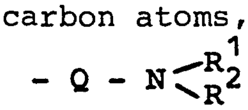

II

in which Q represents an alkylene group having from

1 to 4 carbon atoms, and $R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl substitutent having from 1 to 4 carbon atoms, or

one of W and Z represents a group E - $(CH_2)_n$ - X - $(CH_2)_m$ - NH- as defined above, and the other represents a hydrogen atom; a straight or branched chain alkyl group having from 1 to 6 carbon atoms; a phenyl group; an $-OR^3$ group in which $R^3$ represents a straight or branched chain alkyl group having from 1 to 4 carbon atoms; or an $NR^4R^5$ group in which $R^4$ and $R^5$, which may be the same or different, each represents a hydrogen atom, an unsubstituted or substituted straight or branched chain alkyl group having from 1 to 6 carbon atoms, or a phenyl group;

X represents -O- or -S-;

n represents 0 or 1; and

m represents 2, 3 or 4,

and in which an alkyl group may have a straight or branched chain, and in which an alkyl group, for example, alkyl groups $R^4$ and $R^5$, may be unsubstituted or substituted, by one or more groups selected from

(i) $-OR^6$ groups in which $R^6$ represents a straight or branched chain alkyl group having from 1 to 6 carbon atoms;

(ii) cycloalkyl groups having from 3 to 7 carbon atoms;

(iii) $-NR^7R^8$ groups in which $R^7$ and $R^8$, which may be the same or different, each represents a hydrogen atom, a straight or branched chain alkyl group having from 1 to 4 carbon atoms, a phenyl group or an acyl group having from 1 to 4 carbon atoms in the alkyl moiety, and $R^7$ and $R^8$ together with the nitrogen atom to which they are attached may form a 5 or 6 membered ring;

(iv) $-COOR^9$ groups in which $R^9$ represents a straight or branched chain alkyl group having from 1 to 4 carbon atoms;

(v) $-CONR^7 R^8$ groups in which $R^7$ and $R^8$ are defined above;

(vi) alkylsulphonyl groups having from 1 to 4 carbon atoms in the alkyl moiety and phenylsulphonyl groups;

(vii) cyano and nitro groups;

(viii) phenyl groups which may be substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl moiety having a straight or branched chain and from 1 to 4 carbon atoms;

(ix) aromatic and non-aromatic heterocyclic groups having from 5 to 8 ring members and one or two hetero-atoms selected from oxygen, sulphur and nitrogen atoms, and optionally having an alkyl substitutent having from 1 to 4 carbon atoms on a ring nitrogen atom, for example, a furyl, tetrahydrofuryl, thienyl, pyridyl, dihydropyranyl, pyrrolidinyl, N-lower alkyl-pyrrolidinyl, or piperidyl group,

and in which a phenyl group other than a phenyl group E (which is as defined above), may be unsubstituted or substituted as defined in (viii) above;

and salts of a compound of formula I.

2. A compound of formula XXXI

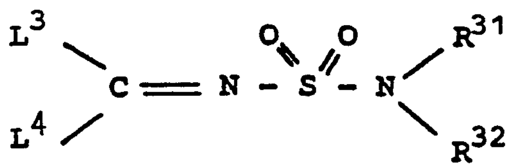

XXXI

in which

$R^{31}$ and $R^{32}$, which may be the same or different, each represents a hydrogen atom, a straight or branched chain alkyl group having from 1 to 4 carbon atoms, a

cycloalkyl group having from 3 to 7 carbon atoms, a phenyl group, or a phenalkyl group having from 1 to 4 carbon atoms in the alkyl moiety,

wherein an alkyl, cycloalkyl, phenyl or phenalkyl group may be unsubstituted or substituted by one or more substitutents, which may be the same or different, for example, by one or two substitutents, especially by one substitutent, selected from halogen atoms, straight or branched chain alkyl groups having from 1 to 4 carbon atoms, straight or branched chain alkoxy groups having from 1 to 4 carbon atoms, and nitro and trifluoromethyl groups; or

$R^{31}$ and $R^{32}$ together with the nitrogen atom to which they are attached, form a 4- to 7-membered ring which may contain one or more further hetero-atoms, for example, selected from nitrogen, oxygen and sulphur atoms;

$L^3$ and $L^4$, which may be the same or different, each represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms,

with the proviso that in $L^3$ and $L^4$, the two radicals $R^{46}$ may be the same or different but the two radicals X must be the same, and

with the exception of those compounds in which $R^{31}$ and $R^{32}$

both represent hydrogen atoms and $L^3$ and $L^4$ both represent alkylthio or alkoxy groups.

3. A compound of formula XXXV

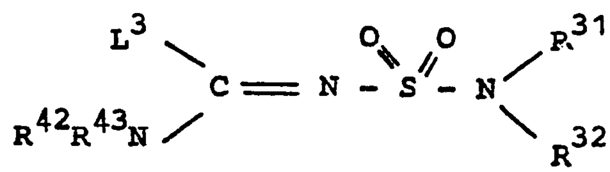

XXXV

in which

$R^{31}$ and $R^{32}$, which may be the same or different, each represents a hydrogen atom, a straight or branched chain alkyl group having from 1 to 4 carbon atoms, a cycloalkyl group having from 3 to 7 carbon atoms, a phenyl group, or a phenalkyl group having from 1 to 4 carbon atoms in the alkyl moiety,

wherein an alkyl, cycloalkyl, phenyl or phenalkyl group may be unsubstituted or substituted by one or more substitutents, which may be the same or different, for example, by one or two substitutents, especially by one substitutent, selected from halogen atoms, straight or branched chain alkyl groups having from 1 to 4 carbon atoms, straight or branched chain alkoxy groups having from 1 to 4 carbon atoms, and nitro and trifluoromethyl groups; or

$R^{31}$ and $R^{32}$ together with the nitrogen atom to which they are attached, form a 4- to 7-membered ring which may contain one or more further hetero-atoms, for example, selected from nitrogen, oxygen and sulphur atoms;

$L^3$ represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two substitutents selected from alkyl,

alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms, and

$R^{42}$ and $R^{43}$, which may be the same or different, each represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

wherein an alkyl, phenyl or phenyalkyl group may be unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups.

4. A compound as claimed in claim 1, wherein E represents a 2-guanidino-4-thiazolyl group or a 3-(1-piperidinylmethyl)phenyl group.

5. A compound as claimed in claim 4, wherein when E represents a 2-guanidino-4-thiazolyl group, n is 1, X is -S-, and m is 2.

6. A compound as claimed in claim 4, wherein when E represents a 3-(1-piperidinylmethyl)phenyl group, n is 0, X is -O-, and m is 3.

7. A compound as claimed in any one of claims 4 to 6, wherein W or Z represents an $NR^4R^5$ group, a methoxy group or a phenyl group.

8. A compound as claimed in claim 1 which is 3-/2-/(2-Guanidino-4-thiazolyl)methylthio/ethyl/amino-5-methylamino-6-methyl-1,2,4,6-thiatriazine-1,1-dioxide.

9. A compound as claimed in claim 1 which is [2-[(2-Guanidino-4-thiazolyl)methylthio]ethyl]amino-5-tert.-butylamino-6-methyl-1,2,4,6-thiatriazine-1,1-dioxide.

10. A compound as claimed in claim 1 which is 3-[3-[3-(1-Piperidinylmethyl)phenoxy]propyl]amino-5-tert.-butylamino-6-methyl-1,2,4,6-thiatriazine-1,1-dioxide.

11. A compound as claimed in claim 1 which is 2-Methyl-3-[2-[(2-guanidino-4-thiazolyl)methylthio]-ethyl]-amino-5-tert.-butylamino-1,2,4,6-thiatriazine-1,1-dioxide and the hydrochloride salt thereof.

12. A compound as claimed in claim 1 which is 2-Methyl-3-[2-[(2-guanidino-4-thiazolyl)methylthio]-ethyl]amino-5-dimethylamino-1,2,4,6-thiatriazine-1,1-dioxide.

13. A compound as claimed in claim 1 which is 2-Methyl-3-[2-[(2-guanidino-4-thiazolyl)methylthio]-ethyl]amino-5-methoxy-1,2,4,6-thiatriazine-1,1-dioxide.

14. A compound as claimed in claim 1 which is 2-Methyl-3-[2-[(2-guanidino-4-thiazolyl)methylthio]-ethyl]amino-5-phenyl-1,2,4,6-thiatriazine-1,1-dioxide.

15. A compound as claimed in claim 1 which is 3-[2-[(2-Guanidino-4-thiazolyl)methylthio]ethyl]amino-5-amino-6-methyl-1,2,4,6-thiatriazine-1,1-dioxide, and the trifluoroacetic acid salt thereof.

16. A compound as claimed in claim 1 which is 2-Methyl-3-[2-[(2-guanidino-4-thiazolyl)methylthio]-ethyl]amino-5-amino-1,2,4,6-thiatriazine-1,1-dioxide.

17. A compound as claimed in claim 1 which is 3-[3-[3-(1-Piperidinylmethyl)phenoxy]propyl]amino-5-amino-6-methyl-1,2,4,6-thiatriazine-1,1-dioxide, and its trifluoroacetic acid salt.

18. A Compound as claimed in claim 1 which is 2-Methyl-3-[3-[3-(1-piperidinylmethyl)phenoxy]-propyl]amino-5-amino-1,2,4,6-thiatriazine-1,1-dioxide, and its trifluoroacetic acid salt.

19. A process for the production of a compound of formula I as claimed in claim 1, which comprises reacting a 1,2,4,6-thiatriazine of formula IIIA or IIIB

IIIA 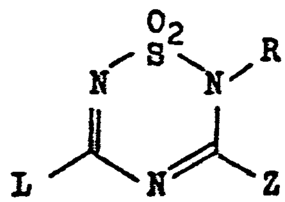 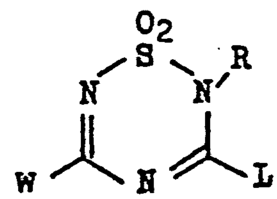 IIIB

in which L represents a halogen atom, an alkoxy, phenoxy or phenylalkoxy group, an alkylthio, phenylthio or phenylalkylthio group, an alkylsulphinyl, phenylsulphinyl or phenylalkylsulphinyl group, or an alkylsulphonyl, phenylsulphonyl or phenylalkylsulphonyl group, in which groups an alkyl group or alkyl moiety has from 1 to 4 carbon atoms, and a phenyl group may be substituted as defined in claim 1, and R, W and Z are as defined in claim 1, with a compound of the general formula IV

$$E-(CH_2)_n-X-(CH_2)_m-NH_2 \qquad IV$$

in which E, X, m and n are as defined for formula I.

20. A process as claimed in claim 19, wherein when Z in compound IIIA or IIIB represents a group N $R^4R^5$ in which $R^4$ represents a protecting group that can be removed by hydrogenolysis or by acid or base catalysed hydrolysis, this group is removed from the resulting compound of formula I by hydrogenolysis or by acid or base catalysed hydrolysis to provide a compound of formula I in which W or Z is a group $-NHR^5$ or $-NH_2$.

21. A process as claimed in claim 19 or claim 20, wherein a compound of formula IIIA in which R is as defined in claim 1, Z represents a group $NHR^4$ in which $R^4$ is as defined in claim 1 and L represents a group $R^{10}$ which represents an alkoxy, phenoxy or phenylalkoxy group, an alkylthio, phenylthio or phenylalkylthio group, is produced by cyclising a compound of the general formula XI

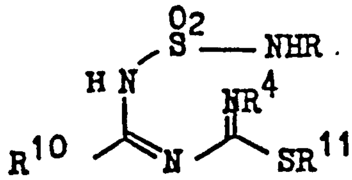

XI

in which R, $R^4$ and $R^{10}$ are as defined above, and $R^{11}$ represents an alkyl or phenalkyl group, by heating.

22. A process as claimed claim 21, wherein a compound of formula XI is produced by reacting a compound of the general formula IX

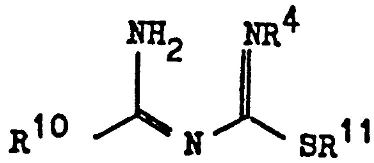

IX

in which $R^4$, $R^{10}$ and $R^{11}$ are as defined in claim 21, with a compound of formula X

$ClSO_2NHR$ X

in which R is as defined in claim 1, in the presence of a tertiary base.

23. A process as claimed in claim 22, wherein a compound of formula IX is produced by reacting a compound of formula VII

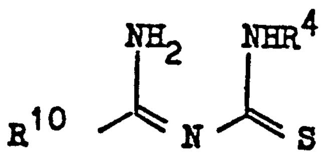

VII

in which $R^4$ and $R^{10}$ are as defined in claim 19, with an alkylating agent which comprises the group $R^{11}$ as defined in claim 21.

24. A process as claimed in claim 23, wherein the alkylating agent has the formula VIII

$R^{11}$-Y VIII

in which $R^{11}$ is as defined in claim 21 and Y represents a halogen atom.

25. A process as claimed in claim 23, wherein a compound of formula VII is produced by reacting a compound of formula V with a compound of formula VI

0159533
HOE 84/S 003K

V 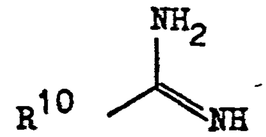 $R^4NCS$ VI

in which formulae $R^4$ and $R^{10}$ are as defined in claim 21.

26. A process as claimed in claim 19, wherein a compound of formula IIIA in which L represents a group $R^{10}$ as defined in claim 21 and Z represents a group $R^{12}$ which represents a hydrogen atom, an alkyl group having up to 6 carbon atoms, a phenyl group or a group $-OR^3$ in which $R^3$ represents a lower alkyl group, is produced by reacting a compound of formula XV

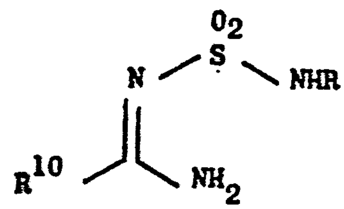 XV

in which R is as defined in claim 19 and $R^{10}$ is as defined in claim 21, with a compound of formula XVI

$R^{12} - C - (OR^3)_3$ XVI

in which $R^{12}$ and $R^3$ are as defined above.

27. A process as claimed in claim 26, wherein a compound of formula XV is produced by reacting a compound of formula XIV

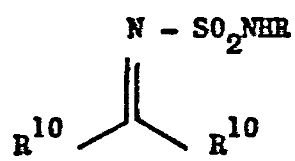 XIV

in which R and $R^{10}$ are as defined in claim 21, with anhydrous ammonia.

28. A process as claimed in claim 27, wherein a compound of formula XIV is produced by reacting a compound of formula XIII

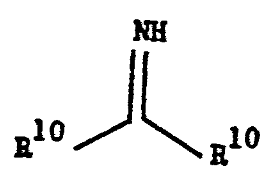 XIII

in which $R^{10}$ is as defined in claim 21, with a compound of formula X

$ClSO_2NHR$ X

in which R is as defined in claim 1.

29. A process as claimed in any one of claims 21 to 28, wherein a resulting compound of formula IIIA in which Z represents a group $R^{10}$ which represents an alkylthio, phenylthio or phenylalkylthio group is converted into a compound of formula in which Z represents an alkylsulphinyl, alkylsulphonyl, phenylsulphinyl, phenylsulphonyl, phenylalkylsulphinyl or phenylalkylsulphonyl group by oxidation of the thio group, for example, using a peracid, hydrogen peroxide, or a periodate, perborate or permanganate salt.

30. A process as claimed in any one of claims 19 to 28, wherein a resulting compound of formula IIIA in which Z represents a group $R^{10}$ which represents an alkylthio or phenylthio group is reacted with an excess of a halogen to give a compound of formula IIIA in which Z represents a halogen atom.

31. A process as claimed in claim 19, wherein a compound of formula IIIA in which L represents a halogen atom and Z represents a group $-NR^4 R^5$ and/or a compound of formula IIIB in which L represents a halogen atom and W represents a group $-NR^4 R^5$ is produced by reacting a compound of formula XXII

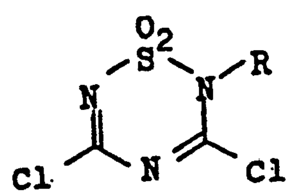

XXII

in which R is as defined in claim 1, with a compound of formula

$HNR^4 R^5$ XXIII

in which $R^4$ and $R^5$ are as defined in claim 1.

32. A process as claimed in claim 31, wherein compound XXII is reacted with the amine XXIII at a temperature

within the range of from 0 to 20°C to give a mixture of compounds IIIA and IIIB.

33. A process as claimed in claim 32, wherein compound XXII is reacted with the amine XXIII at a temperature within the range of from -40 to -20 °C to give predominantly a compound of formula IIIB.

34. A process as claimed in any one of claims 31 to 33, wherein a compound of formula XXII is produced by reacting a compound of formula XXI

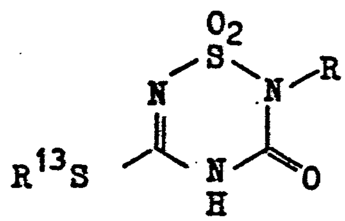

XXI

in which $R^{13}$ represents an alkyl, phenyl or phenalkyl group, with excess phosphorus pentachloride.

35. A pharmaceutical preparation which comprises a compound of the general formula I as claimed in any one of claims 1 and 4 to 18, or a physiologically tolerable salt thereof, in admixture or conjunction with a pharmaceutically suitable carrier.

36. A compound of the general formula I as claimed in any one of claims 1 and 4 to 18, or a physiologically tolerable salt thereof, for the manufacture of a medicament for the treatment of conditions resulting from stimulation of by histamine of histamine H-2 receptors.

37. A method of treating conditions resulting from stimulation by histamine of histamine H-2 receptors in a mammal, which comprises administering to the mammal a compound of formula I as claimed in any one of claims 1 and 4 to 18.

38. A method as claimed in claim 37, wherein there is also administered to the mammal a histamine H-1 antagonist.

39. A compound as claimed in claim 2, wherein $R^{31}$ represents a hydrogen atom, and $R^{32}$ represents a hydrogen atom or an alkyl group having from 1 to 4 carbon atoms.

40. A compound as claimed in claim 2 wherein $R^{31}$ represents a hydrogen atom and $R^{32}$ represents a methyl group.

41. A compound as claimed in claim 2, wherein $R^{31}$ and $R^{32}$ both represent hydrogen atoms.

42. A compound as claimed in claim 2 or any one of claims 39 to 41, wherein $L^3$ represents an alkylthio group having from 1 to 4 carbon atoms, and $L^4$ represents a phenylthio group.

43. A compound as claimed in claim 2 or any one of claims 39 to 42, wherein $L^3$ and $L^4$ both represent substituted phenyl groups.

44. A compound as claimed in claim 43, wherein $L^3$ and $L^4$ both represent mono-substituted phenyl groups.

45. A compound as claimed in claim 2, wherein $R^{31}$ = $R^{32}$ = hydrogen, $L^3$ = methylthio and $L^4$ = phenylthio.

46. A compound as claimed in claim 2, wherein $R^{31}$ = hydrogen, $R^{32}$ = methyl, $L^3$ = methylthio and $L^4$ = phenylthio.

47. A compound as claimed in claim 2, wherein $R^{31}$ = $R^{32}$ = hydrogen, and $L^3$ = $L^4$= 2-nitrophenoxy.

48. A compound as claimed in claim 2, wherein $R^{31}$ = hydrogen, $R^{32}$ = methyl, and $L^3$ = $L^4$ = 2-nitrophenoxy.

49. A compound as claimed in claim 2, having the formula XIV

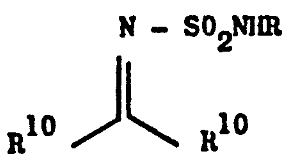

XIV

in which

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4

carbon atoms, straight and branched chain alkoxy groups having from 1 to 4 carbon atoms, nitro and trifluoromethyl groups; and

$R^{10}$ represents an alkoxy, alkylthio, phenoxy, phenylthio, phenylalkoxy or phenylalkylthio group.

50. A compound as claimed in claim 2, having the formula XLVII

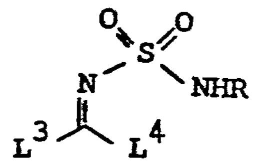

XLVII

in which $L^3$ and $L^4$ are as defined in claim 2 and R is as defined in claim 49.

51. A process for the production of a compound of formula XXXI as defined in claim 2, including those compounds in which $R^{31}$ and $R^{32}$ both represent hydrogen atoms and $L^3$ and $L^4$ both represent alkyloxy or alkylthio groups, which comprises reacting a compound of formula XXXII

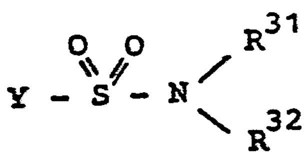

XXXII

in which Y represents a halogen atom, and $R^{31}$ and $R^{32}$ are as defined in claim 2, with a compound of the general formula XXXIII or with a compound of the general formula XXXIV

XXXIII

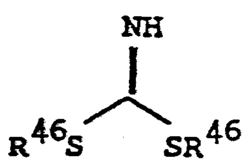

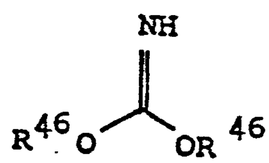

XXXIV

in which $R^{46}$ is as defined in claim 2.

52. A process as claimed in claim 51, wherein in a compound of formula XXXIII,one radical $R^{46}$ represents a methyl group and the other radical $R^{46}$ a phenyl group, and in a compound of formula XXXIV; both radicals $R^{46}$ represent substituted phenyl groups.

53. A compound as claimed in claim 2 for use in the production of sulphamoyl guanidines, sulphamoyl

isothioureas, sulphamoylthioureas and heterocyclic systems.

54. A compound as claimed in claim 3, wherein $R^{42}$ and $R^{43}$ both represent hydrogen atoms, or one represents a hydrogen atom and the other represents a methyl group.

55. A compound as claimed in claim 3 or claim 54, wherein $R^{31}$ represents a hydrogen atom, and $R^{32}$ represents a hydrogen atom or a straight or branched chain alkyl group having from 1 to 6 carbon atoms, especially a methyl or ethyl group.

56. A compound as claimed in claim 3, claim 52 or claim 53, wherein $L^3$ represents a lower alkylthio group, especially a methylthio group, or represents a phenoxy group, especially a substituted phenoxy group.

57. A compound as claimed in claim 3, having the formula XV

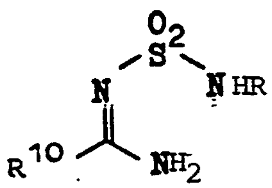

XV

in which

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups; and

$R^{10}$ represents an alkoxy, alkylthio, phenoxy, phenylthio, phenylalkoxy or phenylalkylthio group.

58. A compound as claimed in claim 3, having the formula XLIII

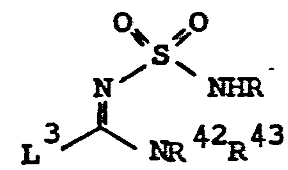

XLIII

in which $L^3$ is as defined in claim 3, and R, $R^{42}$ and $R^{43}$ are as defined for R in claim 57, R, $R^{42}$ and $R^{43}$ being the same or different.

59. A process for the production of a compound of formula XXXV as claimed in claim 3, which comprises reacting a compound of formula XXXI

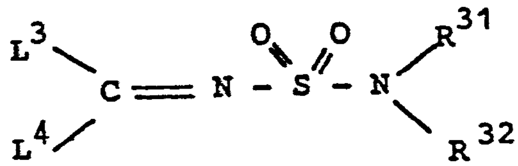

XXXI

in which $R^{31}$, $R^{32}$ and $L^3$ are as defined in claim 3, and $L^4$ is as defined for $L^3$, $L^3$ and $L^4$ being the same or different, with a compound of formula

$HNR^{42}R^{43}$ XXXVI

in which $R^{42}$ and $R^{43}$ are as defined in claim 3.

60. A compound as claimed in claim 3, for use in the production of sulpamoyl guanidines, sulphamoyl isothioureas, sulphamoylthioureas, and heterocyclic systems.

61. A compound of formula IIIA

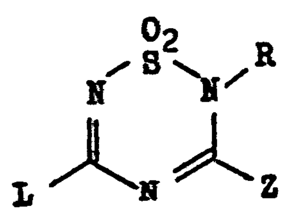

IIIA

in which L, Z and R are as defined in claim 1, Z having any of the meanings given in claim 1.

62. A compound as claimed in claim 61, having the formula XII

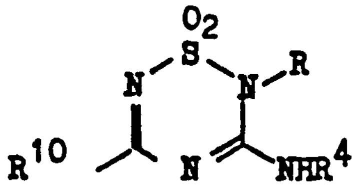

XII

in which R and $R^4$ are as defined in claim 1 and $R^{10}$ is as defined in claim 21.

63. A compound as claimed in claim 61, having the formula XVII

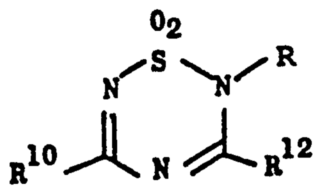

XVII

in which R is as defined in claim 1, $R^{10}$ is as defined in claim 21 ans $R^{12}$ is as defined in claim 26.

64. A compound as claimed in claim 61, having the formula

XVIII

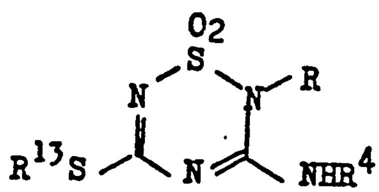

XVIII in which R and $R^4$ are as defined in claim 1 and $R^{13}$ is as defined in claim 34.

65. A compound as claimed in claim 61, having the formula XIX

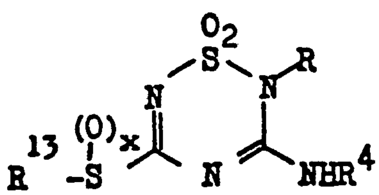

XIX

XIX

in which R and $R^4$ are as defined in claim 1, $R^{13}$ is as defined in claim 34, and x is 1 or 2.

66. A compound as claimed in claim 61, having the formula XX

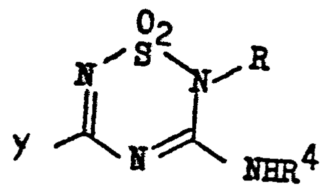

XX

XX

in which R and $R^4$ are as defined in claim 1 and Y is as defined in claim 24.

67. A compound as claimed in claim 61, having the formula XXIV

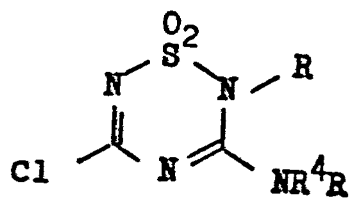

XXIV

in which R, $R^4$ and $R^5$ are as defined in claim 1.

68. A compound having the formula XXV

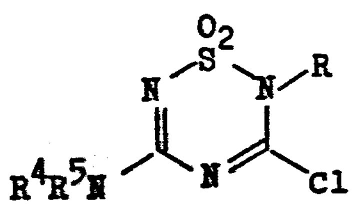

XXV

in which R, $R^4$ and $R^5$ are as defined in claim 1.

69. A compound having the formula XXII

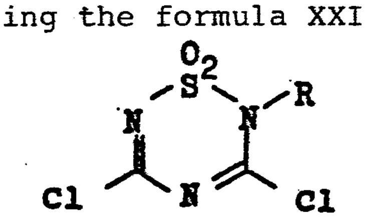

XXII

in which R is as defined in claim 1.

70. The use of a compound as claimed in any one of claims 1 and 4 to 18 for the manufacture of a medicament for the treatment of conditions resulting from stimulation by histamine of histamine H-2 receptors.

HOE 84/S 003K
0159533

Claims for Austria:

1 . A process for the production of a compound of formula I

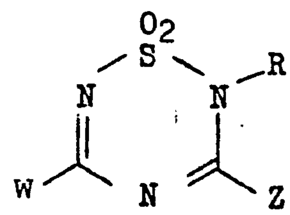

I

in which formula

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

W and Z, which may be the same or different, each represents a group $E-(CH_2)_n - X -(CH_2)_m - NH-$ in which E represents a phenyl, imidazolyl, thiazolyl, furyl, thienyl, or pyridyl radical,

which radicals may be unsubstituted or may have one or two substitutents, selected from straight and branched chain alkyl groups having from 1 to 4 carbon atoms, guanidino and mono-alkyl guanidino groups, the alkyl moiety of which has a straight or branched chain and from 1 to 4 carbon atoms, and groups of the formula II

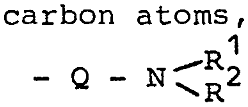

II

in which Q represents an alkylene group having from

1 to 4 carbon atoms, and $R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl substitutent having from 1 to 4 carbon atoms, or

one of W and Z represents a group $E - (CH_2)_n - X - (CH_2)_m - NH-$ as defined above, and the other represents a hydrogen atom; a straight or branched chain alkyl group having from 1 to 6 carbon atoms; a phenyl group; an $-OR^3$ group in which $R^3$ represents a straight or branched chain alkyl group having from 1 to 4 carbon atoms; or an $NR^4R^5$ group in which $R^4$ and $R^5$, which may be the same or different, each represents a hydrogen atom, an unsubstituted or substituted straight or branched chain alkyl group having from 1 to 6 carbon atoms, or a phenyl group;

X represents -O- or -S-;

n represents 0 or 1; and

m represents 2, 3 or 4,

and in which an alkyl group may have a straight or branched chain, and in which an alkyl group, for example, alkyl groups $R^4$ and $R^5$, may be unsubstituted or substituted, by one or more groups selected from

(i) $-OR^6$ groups in which $R^6$ represents a straight or branched chain alkyl group having from 1 to 6 carbon atoms;

(ii) cycloalkyl groups having from 3 to 7 carbon atoms;

(iii) $-NR^7R^8$ groups in which $R^7$ and $R^8$, which may be the same or different, each represents a hydrogen atom, a straight or branched chain alkyl group having from 1 to 4 carbon atoms, a phenyl group or an acyl group having from 1 to 4 carbon atoms in the alkyl moiety, and $R^7$ and $R^8$ together with the nitrogen atom to which they are attached may form a 5 or 6 membered ring;

(iv) $-COOR^9$ groups in which $R^9$ represents a straight or branched chain alkyl group having from 1 to 4 carbon atoms;

(v) $-CONR^7 R^8$ groups in which $R^7$ and $R^8$ are defined above;

(vi) alkylsulphonyl groups having from 1 to 4 carbon atoms in the alkyl moiety and phenylsulphonyl groups;

(vii) cyano and nitro groups;

(viii) phenyl groups which may be substituted by one or two substitutents, which may be the same or different, selected from alkyl, alkoxy, methylenedioxy, phenoxy, halogen, dimethylaminomethyl, trifluoromethyl, nitro, cyano, sulphonic acid, sulphonamide, amino, mono-alkylamino and di-alkylamino groups, an alkyl moiety having a straight or branched chain and from 1 to 4 carbon atoms;

(ix) aromatic and non-aromatic heterocyclic groups having from 5 to 8 ring members and one or two hetero-atoms selected from oxygen, sulphur and nitrogen atoms, and optionally having an alkyl substitutent having from 1 to 4 carbon atoms on a ring nitrogen atom, for example, a furyl, tetrahydrofuryl, thienyl, pyridyl, dihydropyranyl, pyrrolidinyl, N-lower alkyl-pyrrolidinyl, or piperidyl group,

and in which a phenyl group other than a phenyl group E (which is as defined above), may be unsubstituted or substituted as defined in (viii) above;

and salts of a compound of formula I

which comprises reacting a 1,2,4,6-thiatriazine of formula IIIA or IIIB

IIIA 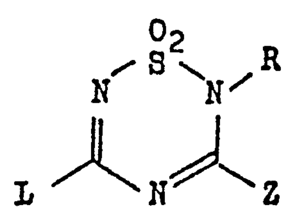 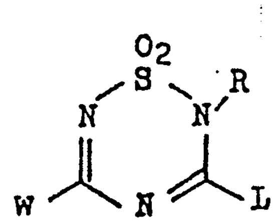 IIIB

in which L represents a halogen atom, an alkoxy, phenoxy

or phenylalkoxy group, an alkylthio, phenylthio or phenylalkylthio group, an alkylsulphinyl, phenylsulphinyl or phenylalkylsulphinyl group, or an alkylsulphonyl, phenylsulphonyl or phenylalkylsulphonyl group, in which groups an alkyl group or alkyl moiety has from 1 to 4 carbon atoms, and a phenyl group may be substituted as defined above, and R, W and Z are as defined above, with a compound of the general formula IV

$$E-(CH_2)_n-X-(CH_2)_m-NH_2 \qquad IV$$

in which E, X, m and n are as defined for formula I.

2. A process as claimed in claim 1, wherein when Z in compound IIIA or IIIB represents a group N $R^4R^5$ in which $R^4$ represents a protecting group that can be removed by hydrogenolysis or by acid or base catalysed hydrolysis, this group is removed from the resulting compound of formula I by hydrogenolysis or by acid or base catalysed hydrolysis to provide a compound of formula I in which W or Z is a group $-NHR^5$ or $-NH_2$.

3. A process as claimed in claim 1 and/or claim 2, wherein a compound of formula IIIA in which R is as defined in claim 1, Z represents a group $NHR^4$ in which $R^4$ is as defined in claim 1 and L represents a group $R^{10}$ which represents an alkoxy, phenoxy or phenylalkoxy group, an alkylthio, phenylthio or phenylalkylthio group, is produced by cyclising a compound of the general formula XI

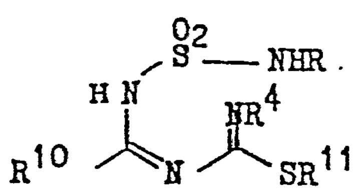

XI

in which R, $R^4$ and $R^{10}$ are as defined above, and $R^{11}$ represents an alkyl or phenalkyl group, by heating.

4. A process as claimed claim 3, wherein a compound of formula XI is produced by reacting a compound of the general formula IX

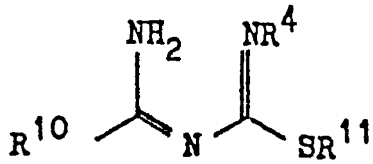

IX

in which $R^4$, $R^{10}$ and $R^{11}$ are as defined in claim 3, with a compound of formula X

$ClSO_2NHR$ X

in which R is as defined in claim 1, in the presence of a tertiary base.

5. A process as claimed in claim 4, wherein a compound of formula IX is produced by reacting a compound of formula VII

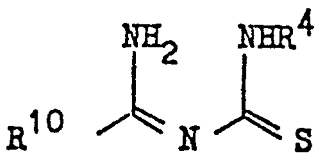

VII

in which $R^4$ and $R^{10}$ are as defined above , with an alkylating agent which comprises the group $R^{11}$ as defined above

6. A process as claimed in claim 5, wherein the alkylating agent has the formula VIII

$R^{11}$-Y VIII

in which $R^{11}$ is as defined in claim 21 and Y represents a halogen atom.

7. A process as claimed in claim 5, wherein a compound of formula VII is produced by reacting a compound of formula V with a compound of formula VI

V 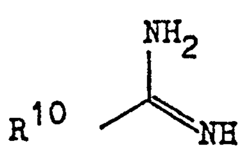 $R^4NCS$ VI

in which formulae $R^4$ and $R^{10}$ are as defined in claim 3,

8. A process as claimed in claim 1 , wherein a compound of formula IIIA in which L represents a group $R^{10}$ as defined in claim 3 and Z represents a group $R^{12}$ which

represents a hydrogen atom, an alkyl group having up to 6 carbon atoms, a phenyl group or a group $-OR^3$ in which $R^3$ represents a lower alkyl group, is produced by reacting a compound of formula XV

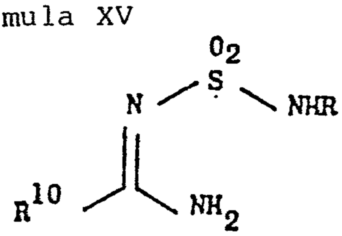

XV

in which R is as defined in claim 19 and $R^{10}$ is as defined in claim 3, with a compound of formula XVI

$$R^{12} - C - (OR^3)_3 \qquad XVI$$

in which $R^{12}$ and $R^3$ are as defined above.

9. A process as claimed in claim 8, wherein a compound of formula XV is produced by reacting a compound of formula XIV

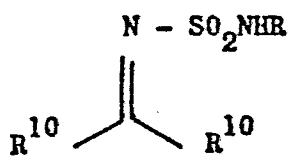

XIV

in which R and $R^{10}$ are as defined in claim 3, with anhydrous ammonia.

10. A process as claimed in claim 9, wherein a compound of formula XIV is produced by reacting a compound of formula XIII

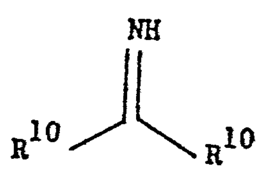

XIII

in which $R^{10}$ is as defined in claim 3, with a compound of formula X

$$ClSO_2NHR \qquad X$$

in which R is as defined in claim 1.

11. A process as claimed in any one of claims 3 to 10, wherein a resulting compound of formula IIIA in which Z represents a group $R^{10}$ which represents an alkylthio, phenylthio or phenylalkylthio group is converted into a

compound of formula in which Z represents an alkylsulphinyl, alkylsulphonyl, phenylsulphinyl, phenylsulphonyl, phenylalkylsulphinyl or phenylalkylsulphonyl group by oxidation of the thio group, for example, using a peracid, hydrogen peroxide, or a periodate, perborate or permanganate salt.

12. A process as claimed in any one of claims 1 to 10, wherein a resulting compound of formula IIIA in which Z represents a group $R^{10}$ which represents an alkylthio or phenylthio group is reacted with an excess of a halogen to give a compound of formula IIIA in which Z represents a halogen atom.

13. A process as claimed in claim 1, wherein a compound of formula IIIA in which L represents a halogen atom and Z represents a group $-NR^4R^5$ and/or a compound of formula IIIB in which L represents a halogen atom and W represents a group $-NR^4R^5$ is produced by reacting a compound of formula XXII

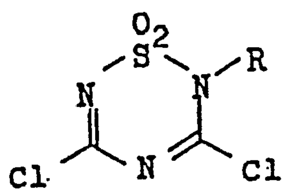

XXII

in which R is as defined in claim 1, with a compound of formula

$HNR^4R^5$ XXIII

in which $R^4$ and $R^5$ are as defined in claim 1.

14. A process as claimed in claim 13, wherein compound XXII is reacted with the amine XXIII at a temperature within the range of from 0 to 20°C to give a mixture of compounds IIIA and IIIB.

15. A process as claimed in claim 14, wherein compound XXII is reacted with the amine XXIII at a temperature within the range of from -40 to -20 °C to give predominantly a compound of formula IIIB.

16. A process as claimed in any one of claims 13 to 15, wherein a compound of formula XXII is produced by reacting

a compound of formula XXI

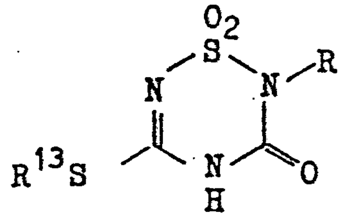

XXI

in which $R^{13}$ represents an alkyl, phenyl or phenalkyl group, with excess phosphorus pentachloride.

17. A process for the production of a pharmaceutical preparation which comprises admixing or conjoining a compound of the formula I as prepared according to any one of claims 1 to 16, or a physiologically tolerable salt thereof with a pharmaceutically suitable carrier.

18. A process as claimed in claim 1, wherein E represents a 2-guanidino-4-thiazolyl group or a 3-(1-piperidinylmethyl)phenyl group.

19. A process as claimed in claim 18, wherein when E represents a 2-guanidino-4-thiazolyl group, n is 1, X is -S-, and m is 2.

20. A process as claimed in claim 18, wherein when E represents a 3-(1-piperidinylmethyl)phenyl group, n is 0, X is -O-, and m is 3.

21. A process as claimed in any one of claims 18 to 20, wherein W or Z represents an $NR^4R^5$ group, a methoxy group or a phenyl group.

22. A process as claimed in claim 1 according to which 3-/2-/(2-guanidino-4-thiazolyl)methylthio7ethyl7amino-5-methylamino-6-methyl-1,2,4,6-thiatriazine-1,1-dioxide is produced.

23. A process as claimed in claim 1 according to which /2-/(2-guanidino-4-thiazolyl)methylthio7ethyl7amino-5-tert.-butylamino-6-methyl-1,2,4,6-thiatriazine-1,1-dioxide is produced.

24. A process as claimed in claim 1 according to which 3-/3-/3-(1-piperidinylmethyl)phenoxy7propyl7amino-5-tert.-butylamino-6-methyl-1,2,4,6-thiatriazine-1,1-dioxide is produced.

25. A process as claimed in claim 1 according to which 2-methyl-3-[2-[(2-guanidino-4-thiazolyl)methylthio]-ethyl]-amino-5-tert.-butylamino-1,2,4,6-thiatriazine-1,1-dioxide and the hydrochloride salt thereof is produced.

26. A process as claimed in claim 1 according to which 2-methyl-3-[2-[(2-guanidino-4-thiazolyl)methylthio]-ethyl]amino-5-dimethylamino-1,2,4,6-thiatriazine-1,1-dioxide.is produced.

27. A process as claimed in claim 1 according to which 2-methyl-3-[2-[(2-guanidino-4-thiazolyl)methylthio]-ethyl]amino-5-methoxy-1,2,4,6-thiatriazine-1,1-dioxide is produced.

28. A process as claimed in claim 1 according to which 2-methyl-3-[2-[(2-guanidino-4-thiazolyl)methylthio]-ethyl]amino-5-phenyl-1,2,4,6-thiatriazine-1,1-dioxide is produced.

29. A process as claimed in claim 1 according to which 3-[2-[(2-guanidino-4-thiazolyl)methylthio]ethyl]amino-5-amino-6-methyl-1,2,4,6-thiatriazine-1,1-dioxide, and the trifluoroacetic acid salt thereof is produced.

30. A process as claimed in claim 1 according to which 2-methyl-3-[2-[(2-guanidino-4-thiazolyl)methylthio]-ethyl]amino-5-amino-1,2,4,6-thiatriazine-1,1-dioxide is produced.

31. A process as claimed in claim 1 according to which 3-[3-[3-(1-piperidinylmethyl)phenoxy]propyl]amino-5-amino-6-methyl-1,2,4,6-thiatriazine-1,1-dioxide, and its trifluoroacetic acid salt is produced.

32. A process as claimed in claim 1 according to which 2-methyl-3-[3-[3-(1-piperidinylmethyl)phenoxy]-propyl]amino-5-amino-1,2,4,6-thiatriazine-1,1-dioxide, and its trifluoroacetic acid salt is produced.

33. A process for the production of a compound of formula XXXI

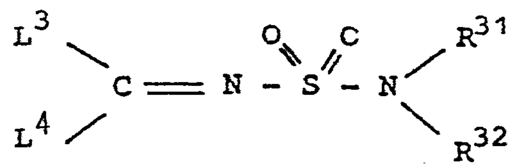

XXXI

in which

$R^{31}$ and $R^{32}$, which may be the same or different, each represents a hydrogen atom, a straight or branched chain alkyl group having from 1 to 4 carbon atoms, a cycloalkyl group having from 3 to 7 carbon atoms, a phenyl group, or a phenalkyl group having from 1 to 4 carbon atoms in the alkyl moiety,

wherein an alkyl, cycloalkyl, phenyl or phenalkyl group may be unsubstituted or substituted by one or more substitutents, which may be the same or different, for example, by one or two substitutents, especially by one substitutent, selected from halogen atoms, straight or branched chain alkyl groups having from 1 to 4 carbon atoms, straight or branched chain alkoxy groups having from 1 to 4 carbon atoms, and nitro and trifluoromethyl groups; or

$R^{31}$ and $R^{32}$ together with the nitrogen atom to which they are attached, form a 4- to 7-membered ring which may contain one or more further hetero-atoms, for example, selected from nitrogen, oxygen and sulphur atoms;

$L^3$ and $L^4$, which may be the same or different, each represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms,

with the proviso that in $L^3$ and $L^4$, the two radicals $R^{46}$ may be the same or different but the two radicals X must be the same,

both represent hydrogen atoms and $L^3$ and $L^4$ both represent alkylthio or alkoxy groups, which

comprises reacting a compound of formula XXXII

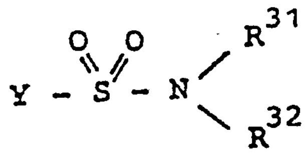

XXXII

in which Y represents a halogen atom, and $R^{31}$ and $R^{32}$ are as defined above, with a compound of the general formula XXXIII or with a compound of the general formula XXXIV

XXXIII

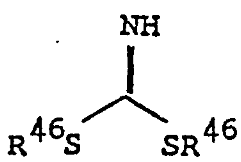

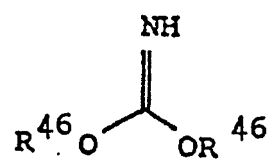

XXXIV

in which $R^{46}$ is as defined above.

34. A process as claimed in claim 33, wherein in a compound of formula XXXIII, one radical $R^{46}$ represents a methyl group and the other radical $R^{46}$ a phenyl group, and in a compound of formula XXXIV; both radicals $R^{46}$ represent substituted phenyl groups.

35. A process as claimed in claim 33, wherein $R^{31}$ represents a hydrogen atom, and $R^{32}$ represents a hydrogen atom or an alkyl group having from 1 to 4 carbon atoms.

36. A process as claimed in claim 33, wherein $R^{31}$ represents a hydrogen atom and $R^{32}$ represents a methyl group.

37. A process as claimed in claim 33, wherein $R^{31}$ and $R^{32}$ both represent hydrogen atoms.

38. A process as claimed in claim 33, or any one of claims 35 to 37, wherein $L^3$ represents an alkylthio group having from 1 to 4 carbon atoms, and $L^4$ represents a phenylthio group.

39. A process as claimed in claim 33, or any one of claims 35 to 38, wherein $L^3$ and $L^4$ both represent substituted phenyl groups.

40. A process as claimed in claim 39, wherein $L^3$ and $L^4$ both represent mono-substituted phenyl groups.

41. A process as claimed in claim 33, wherein $R^{31}$ = $R^{32}$ = hydrogen, $L^3$ = methylthio and $L^4$ = phenylthio.

42. A process as claimed in claim 33, wherein $R^{31}$ = hydrogen, $R^{32}$ = methyl, $L^3$ = methylthio and $L^4$ = phenylthio.

43. A process as claimed in claim 33, wherein $R^{31}$ = $R^{32}$ = hydrogen, and $L^3$ = $L^4$= 2-nitrophenoxy.

44. A process as claimed in claim 33, wherein $R^{31}$ = hydrogen, $R^{32}$ = methyl, and $L^3$ = $L^4$ = 2-nitrophenoxy.

45. A process as claimed in claim 33, in which a compound is produced which has the formula XIV

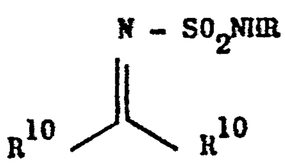

XIV

in which

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4

carbon atoms, straight and branched chain alkoxy groups having from 1 to 4 carbon atoms, nitro and trifluoromethyl groups; and

$R^{10}$ represents an alkoxy, alkylthio, phenoxy, phenylthio, phenylalkoxy or phenylalkylthio group.

46. A process as claimed in claim 33, according to which a compound having the formula XLVII

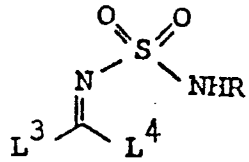

XLVII

in which $L^3$ and $L^4$ are as defined above is produced.

47. A process for the production of a compound of formula XXXV

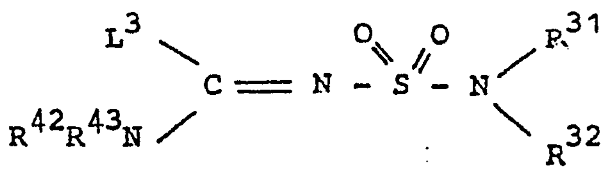

XXXV

in which

$R^{31}$ and $R^{32}$, which may be the same or different, each represents a hydrogen atom, a straight or branched chain alkyl group having from 1 to 4 carbon atoms, a cycloalkyl group having from 3 to 7 carbon atoms, a phenyl group, or a phenalkyl group having from 1 to 4 carbon atoms in the alkyl moiety,

wherein an alkyl, cycloalkyl, phenyl or phenalkyl group may be unsubstituted or substituted by one or more substitutents, which may be the same or different, for example, by one or two substitutents, especially by one substitutent, selected from halogen atoms, straight or branched chain alkyl groups having from 1 to 4 carbon atoms, straight or branched chain alkoxy groups having from 1 to 4 carbon atoms, and nitro and trifluoromethyl groups; or

$R^{31}$ and $R^{32}$ together with the nitrogen atom to which they are attached, form a 4- to 7-membered ring which

may contain one or more further hetero-atoms, for example, selected from nitrogen, oxygen and sulphur atoms;

$L^3$ represents a group $-XR^{46}$ in which X represents an oxygen or sulphur atom and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety,

wherein a phenyl or phenalkyl group may be substituted by one or two substitutents selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms, and

$R^{42}$ and $R^{43}$, which may be the same or different, each represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

wherein an alkyl, phenyl or phenyalkyl group may be unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups, which comprises reacting a compound of formula XXXI

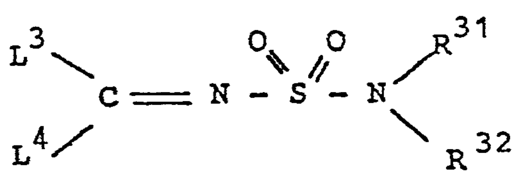

XXXI

in which $R^{31}$, $R^{32}$ and $L^3$ are as defined in above and $L^4$ is as defined for $L^3$, $L^3$ and $L^4$ being the same or different, with a compound of formula

$$HNR^{42}R^{43} \quad XXXVI$$

in which $R^{42}$ and $R^{43}$ are as defined above.

48. A process as claimed in claim 47, wherein $R^{42}$ and $R^{43}$ both represent hydrogen atoms, or one represents a hydrogen atom and the other represents a methyl group.

49. A process as claimed in claim 47 or claim 48, wherein $R^{31}$ represents a hydrogen atom, and $R^{32}$ represents a hydrogen atom or a straight or branched chain alkyl group having from 1 to 6 carbon atoms, especially a methyl or ethyl group.

50. A process as claimed in claim 47, wherein $L^3$ represents a lower alkylthio group, especially a methylthio group, or represents a phenoxy group, especially a substituted phenoxy group.

51. A process as claimed in claim 47, which produces a compound having the formula XV

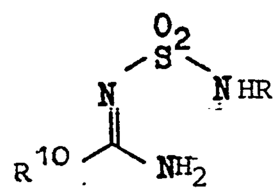

XV

in which

R represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and

trifluoromethyl groups; and
$R^{10}$ represents an alkoxy, alkylthio, phenoxy, phenylthio, phenylalkoxy or phenylalkylthio group.

52. A process as claimed in claim 47, according to which a a compound having the formula XLIII

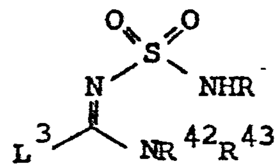

XLIII

in which $L^3$ is as defined above , and R, $R^{42}$ and $R^{43}$ are as defined for R in claim 51, R, $R^{42}$ and $R^{43}$ being the same or different is produced.

53. A process according to claim 1 according to which a compound of formula IIIA

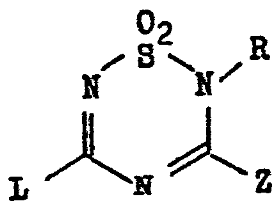

IIIA

in which L, Z and R are as defined in claim 1, Z having any of the meanings given in claim 1 is produced.

54. A process as claimed in claim 1, according to which a compound having the formula XII

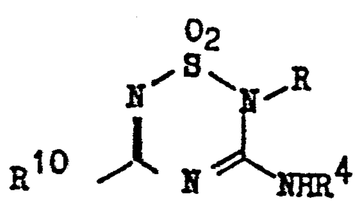

XII

in which R, R and R are as defined above is produced.

55. A process as claimed in claim 1, according to which a compound having the formula XVII

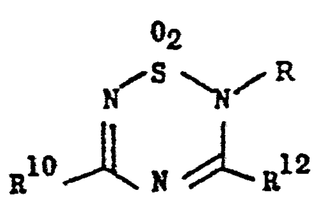

XVII

in which R, $R^{10}$, $R^{12}$ are as defined above.

56. A process as claimed in claim 1, according to which a compound having the formula

XVIII 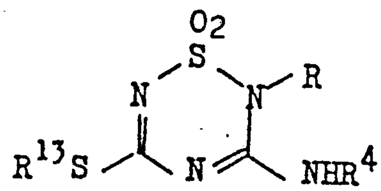

in which R, $R^4$ and $R^{13}$ are as defined above is produced.

57. A process as claimed in claim 1, according to which a compound having the formula XIX

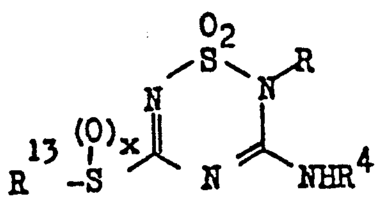 XIX XIX

in which R, $R^4$ and $R^{13}$ are as defined above, and x is 1 or 2.

58. A process as claimed in claim 1, according to which a compound having the formula XX

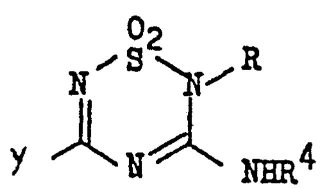 XX

in which R, $R^4$ and Y are as defined above.

59. A process as claimed in claim 1, according to which a compound having the formula XXIV

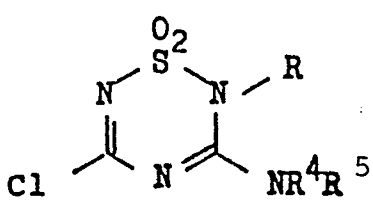 XXIV

in which R, $R^4$ and $R^5$ are as defined above is produced.

60. A process according to claim 1 according to which a compound having the formula XXV

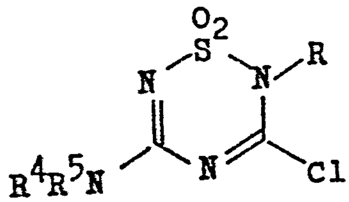

XXV

in which R, $R^4$ and $R^5$ are as defined in claim 1 is produced.

61. A process according to claim 1 wherein a compound having the formula XXII

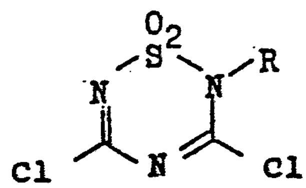

XXII

in which R is as defined in claim 1 is produced.